# EUROPEAN PATENT APPLICATION

(11) **EP 4 516 801 A1**
(43) Date of publication of application: **05.03.2025**
(21) Application number: 23796509.0
(22) Date of filing: 27.04.2023
(51) Int. Cl.: C07K 2/00

(54) **COMPOUND AND USE OF SAME**

(30) Priority: 27.04.2022 JP 2022073764
(71) Applicant: Japan Science and Technology Agency, Kawaguchi-shi, Saitama 332-0012 (JP)
(72) Inventor: MURAOKA Takahiro, Fuchu-shi, Tokyo 183-8538 (JP); UCHIDA Noriyuki, Fuchu-shi, Tokyo 183-8538 (JP); FU Jakuei, Fuchu-shi, Tokyo 183-8538 (JP); HIGUCHI Genki, Fuchu-shi, Tokyo 183-8538 (JP)
(74) Representative: Vossius & Partner Patentanwälte Rechtsanwälte mbB
(86) International application number: PCT/JP2023/016741
(87) International publication number: WO 2023/210774

(57) **Abstract**

Provided is a compound that includes a peptide structure represented by Formula (p1) or (p2). Also provided is a method for producing droplets using the compound. Xa is an amino acid residue having a hydrophilic side chain; Xb is an amino acid residue having a side chain represented by Formula (b1); Xc and Xd are each independently an amino acid residue or a glycine residue having a hydrophobic side chain; and n is an integer of 1 to 3. Ar¹ is an aryl group or heteroaryl group which may have a substituent; and Ar² is an arylene group or a heteroarylene group which may have a substituent.

XaXb(XaXc)ₙXaXdXa... (p1)

XaXd(XaXc)ₙXaXbXa... (p2)

* -Ar²-N=N-Ar¹ (b1)

## Description

### TECHNICAL FIELD

The present invention relates to a compound and use of the same. In particular, the present invention relates to a compound, a gelling agent, a droplet control agent, a droplet control method, a droplet production kit, a droplet production method, a droplet formation composition, and a reaction method.

Priority is claimed on Japanese Patent Application No. 2022-073764, filed April 27, 2022, the content of which is incorporated herein by reference.

### BACKGROUND ART

Sensing for targeting single molecules (single molecule analysis) is a digital analysis technology that gives a signal of "0 or 1" rather than an average value and has thus attracted attention in relation to searches for drug candidate substances and the like. Single molecule sensing requires a reaction to label a single target molecule and a reaction to amplify the molecule. In general, to perform a chemical reaction, it is necessary to localize and mix a substrate and a reactant in a fluid solution space at sufficient concentrations. Accordingly, to perform a reaction starting from a single molecule, a technology is required to localize and mix substrate molecules and reactant molecules in an extremely small fluid space.

One example of a single molecule sensing technology put to practical use is digital ELISA. Digital ELISA utilizes an antigen-antibody reaction using an antibody that selectively bonds to a target with high affinity, making detection possible in a reaction space (reaction solution) of 1 fL (1 × 10⁻¹⁵ L) (Non-Patent Document 1). When an antibody that specifically recognizes the target is available, it is possible to use the digital ELISA method. However, when the target is an unknown compound, a new compound, a trace compound, an unstable compound, or the like, it is difficult to produce an antibody and difficult to use digital ELISA.

When it becomes possible to label a single target molecule with a reaction agent having low affinity, it will be possible to dramatically expand the use of the digital ELISA method. Therefore, it is considered that the digital ELISA method will be used for new analysis technologies, in particular, in life science research and drug discovery-related industries.

To efficiently and reliably label a single substrate molecule using a low-affinity labeling agent, it is necessary to construct a reaction space concentrated to the same level or greater than the conditions for an antigen-antibody reaction. As a base material therefor, liquid-liquid phase-separated droplets that provide a closed space that maintains fluidity in water are useful. For example, by using droplets having a diameter of 10 nm, it is possible to construct an ultra-small reaction space of 0.5 yL (0.5 x 10⁻²⁴ L). It is possible to produce a large number of droplets cheaply and extremely simply by using a two-phase system of dextran (DEX) and polyethylene glycol (PEG) or the like. However, generally, droplets are unstable and droplets spontaneously fuse together within a few minutes, changing to macro-scale phase separation.

### Citation List

### Non Patent Document

Non Patent Document 1: Y. Rondelez, et al., Microfabricated arrays of femtoliter chambers allow single molecule enzymology. Nat. Nanotech. 2005, 3, 361-365

### SUMMARY OF INVENTION

### Technical Problem

To use droplets as ultrafine reaction sites, a technology is required to stabilize the droplets for a time sufficient for the desired reaction. However, a technology that enables such stabilization has not been developed.

In addition, a gelling agent that is able to reversibly control a gel-sol transition using a simple method is useful for applications such as medical materials.

The present disclosure provides a compound able to be used to control liquid-liquid phase-separated droplets, a gelling agent including the compound, a droplet control agent including the compound, and a droplet production kit. In addition, the present disclosure also provides a method for producing droplets using the compound, a droplet formation composition, a method for controlling droplets, and a reaction using the droplets.

### Solution to Problem

The present disclosure includes the following aspects.
[1] A compound including a peptide structure represented by General Formula (p1) or (p2).

   XaXb(XaXc)ₙXaXdXa... (p1)

   XaXd(XaXc)ₙXaXbXa... (p2)

   [In the formulas, Xa represents an amino acid residue having a hydrophilic side chain, Xb represents an amino acid residue having a side chain represented by General Formula (b1), Xc and Xd each independently represent an amino acid residue or a glycine residue having a hydrophobic side chain, n represents an integer of 1 to 3, a plurality of Xa's may each be identical or different, and when n is 2 or 3, a plurality of Xc's may each be identical or different.]

   *-Ar²-N=N-Ar¹ (b1)

   [In the formula, Ar¹ represents an aryl group or a heteroaryl group which may have a substituent, Ar² represents an arylene group or a heteroarylene group which may have a substituent, and * represents a bond.]
[2] The compound according to [1], in which Xc represents an alanine residue or a glycine residue, and Xd represents an amino acid residue having a hydrophobic side chain having 3 or more carbon atoms.
[3] The compound according to [1] or [2], in which the side chain represented by General Formula (b1) is a side chain represented by General Formula (b1-1). 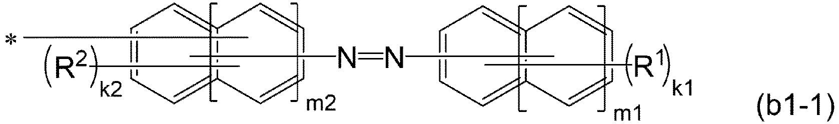 [In the formula, m1 and m2 each independently represent an integer of 0 to 3, R¹ and R² each independently represent a substituent, k1 and k2 each independently represent an integer of 0 or more as allowed by an atomic valence, when k1 is an integer of 2 or more, two or more R¹'s may each be identical or different, when k2 is an integer of 2 or more, the two or more R²'s may each be identical or different, and * represents a bond.]
[4] The compound according to any one of [1] to [3], in which the peptide structure is a peptide structure represented by General Formula (p1-1) or (p2-1). 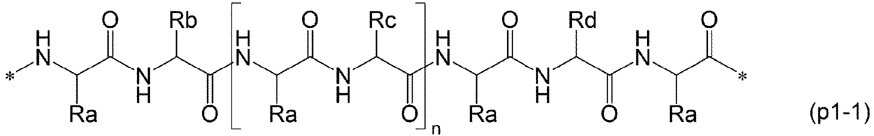 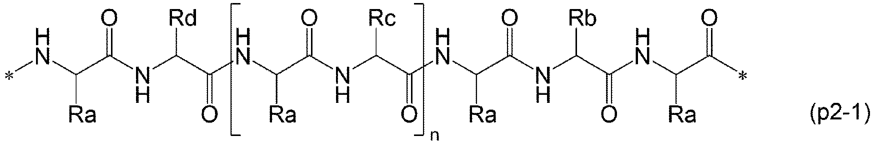 [In the formulas, Ra represents a hydrophilic side chain, Rb represents a side chain represented by General Formula (b1), Rc and Rd each independently represent a hydrophobic side chain or a hydrogen atom, n represents an integer of 1 to 3, a plurality of Ra's may each be identical or different, when n is 2 or 3, the plurality of Ra's may each be identical or different, and * represents a bond.]
[5] The compound according to [4], in which Rc represents a hydrogen atom or a methyl group, and Rd represents a hydrophobic side chain having 3 or more carbon atoms.
[6] The compound according to any one of [1] to [5], which is a peptide consisting of 40 or fewer amino acid residues.
[7] The compound according to any one of [1] to [6], further including a peptide structure represented by Formula (p1-1-1), (p2-1-1), (p1-1-2), or (p2-1-2). 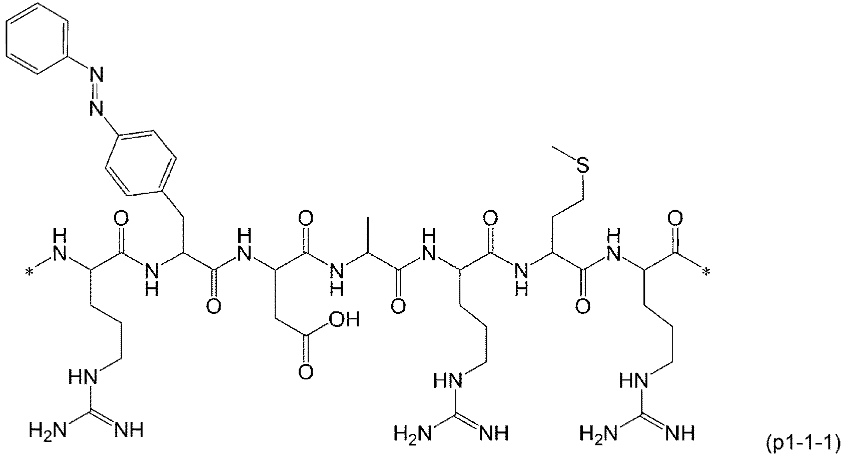 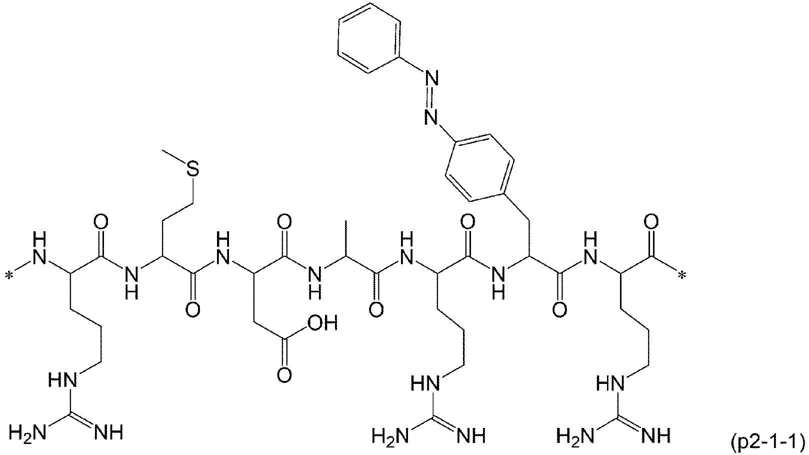 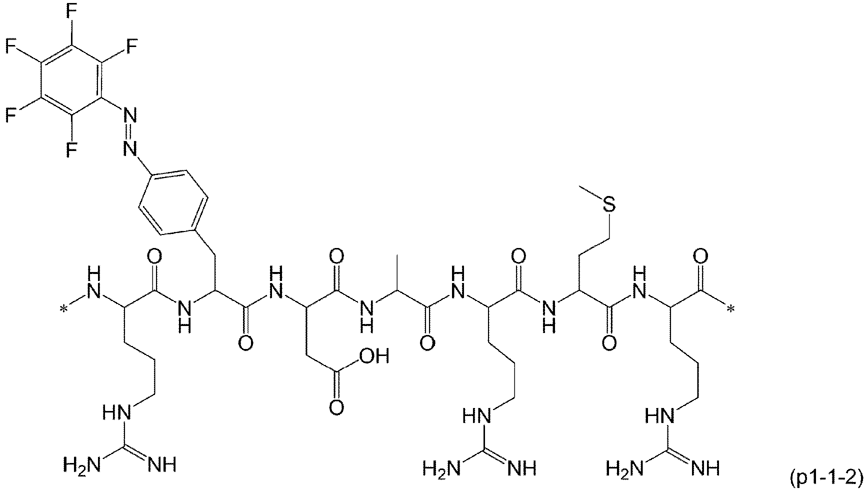 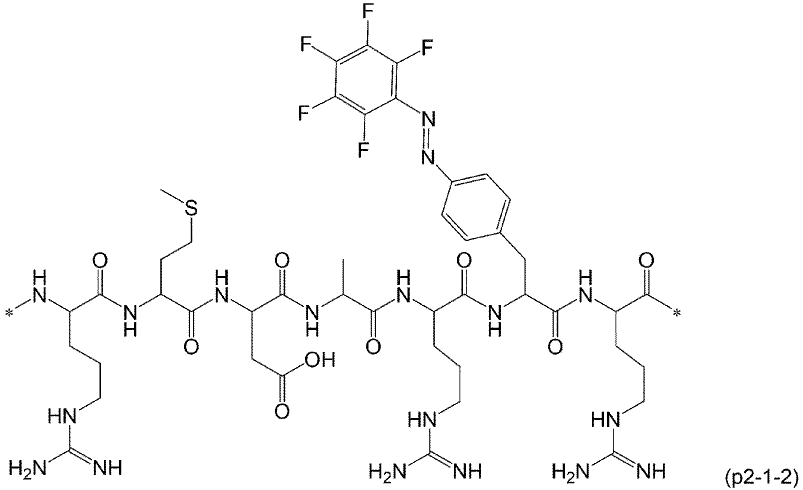 [* is a bond bonded to an adjacent atom.]
[8] The compound according to [1], which is represented by any of Formulas (P1) to (P4). 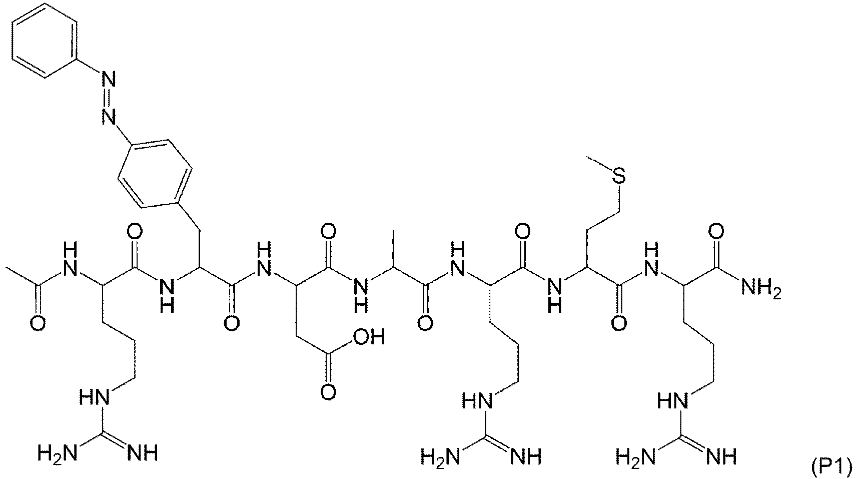 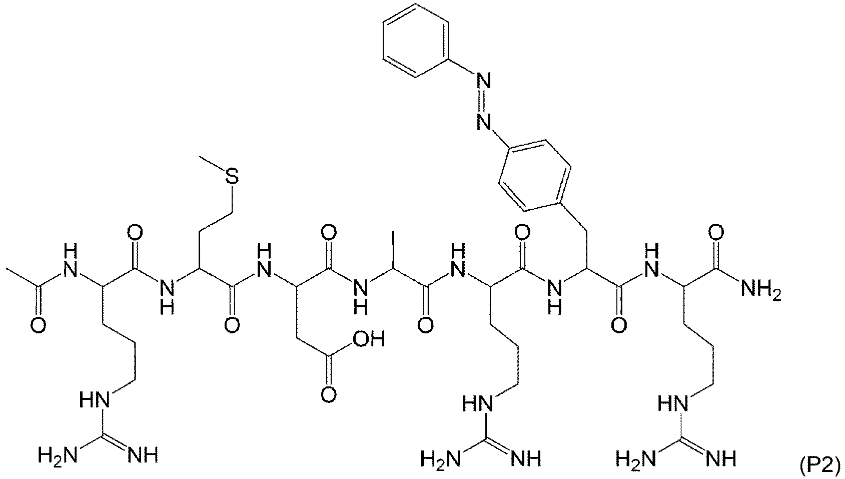 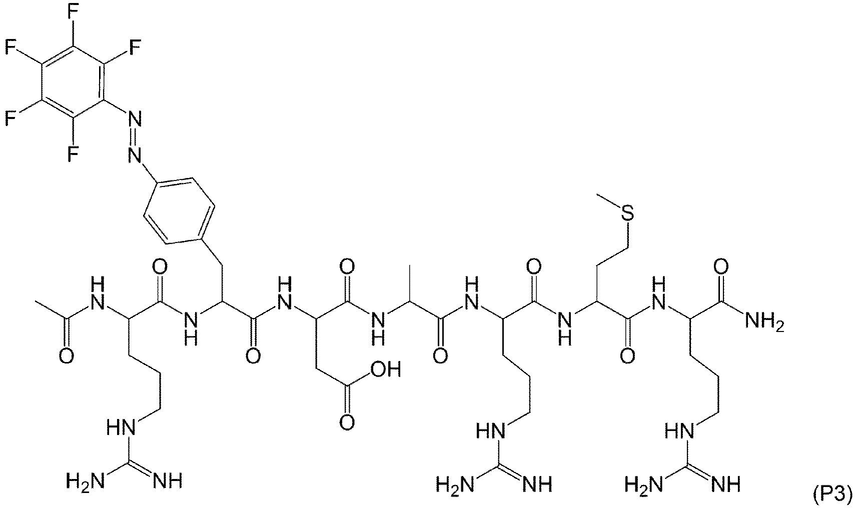 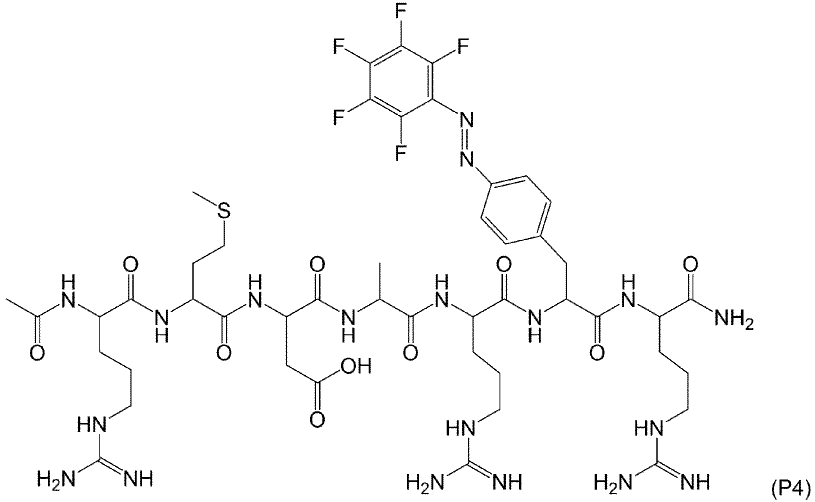
[9] A gelling agent including the compound according to any one of [1] to [8].
[10] A droplet production kit including the compound according to any one of [1] to [8], a polysaccharide, and a polyether.
[11] A method for producing droplets, the method including forming droplets by stirring a solution including the compound according to any one of [1] to [8], a polysaccharide, and a polyether.
[12] The method for producing droplets according to [11], the method further including irradiating the formed droplets with light.
[13] A reaction method including (a) a step of stirring a solution including the compound according to any one of [1] to [8], a polysaccharide, a polyether, and a first reactant to produce a first droplet including the first reactant, (b) a step of stirring a solution including the compound according to any one of [1] to [8], a polysaccharide, a polyether, and a second reactant to produce a second droplet including the second reactant, (c) a step of mixing the first droplet and the second droplet, and (d) a step of irradiating the mixed first droplet and second droplet with light to fuse the first droplet and the second droplet to produce a reaction site droplet including the first reactant and the second reactant.
[14] The reaction method according to [13], further including (e) a step of incubating the reaction site droplet.
[15] A droplet formation composition including the compound according to any one of [1] to [8], a polysaccharide, and a polyether.
[16] A droplet control agent including the compound according to any one of [1] to [8].
[17] A method for controlling droplets, the method including causing droplets formed by stirring a solution including a polysaccharide and a polyether to coexist with the compound according to any one of [1] to [8].
[18] The method for controlling droplets according to [17], the method further including irradiating the droplets coexisting with the compound with light.

### Advantageous Effects of Invention

According to the present disclosure, a compound able to be used to control liquid-liquid phase-separated droplets, a gelling agent including the compound, a droplet control agent including the compound, and a droplet production kit are provided. In addition, according to the present disclosure, a method for producing droplets using the compound, a droplet formation composition, a method for controlling droplets, and a reaction using the droplets are provided.

### BRIEF DESCRIPTION OF DRAWINGS

[FIG. 1] A schematic diagram for explaining a method for producing liquid-liquid phase-separated droplets according to one embodiment.
[FIG. 2] A schematic diagram showing one embodiment of step (c) and step (d) in a reaction method.
[FIG. 3] Results of analysis of a compound (P1) synthesized in Example 1 using matrix-assisted laser desorption/ionization (MALDI).
[FIG. 4] Results of investigating the photoresponsiveness of the compound (P1). (A) shows the absorption spectrum when irradiated with 350 nm light. (B) shows the CD spectrum when irradiated with 350 nm light. (C) shows the absorption spectrum when irradiated with 450 nm light. (D) shows the CD spectrum when irradiated with 450 nm light.
[FIG. 5] A phase-contrast microscope image and a fluorescence microscope image of droplets produced in Example 1. The scale bar indicates 50 µm.
[FIG. 6] Phase-contrast microscope images of the droplets produced in Example 1. The images were taken immediately after (0 hours) and 24 hours after droplet production. The scale bar indicates 100 µm.
[FIG. 7] Phase-contrast microscope images of the droplets produced in Example 1. The images were taken immediately after (0 days) and 16 days after droplet production. The scale bar indicates 100 µm.
[FIG. 8] Microscope images of droplets produced without using the compound (P1). The images were taken 1 minute and 5 minutes after droplet production. The white arrow indicates a large droplet formed by the fusing together of the droplets. The scale bar indicates 100 µm.
[FIG. 9] Fusion of droplets by position-selective light irradiation of the droplets produced in Example 1. The shown microscope images were taken immediately after the start of light irradiation and 6 minutes after the start of light irradiation. The portion surrounded by the dotted circle is the light irradiation spot. The scale bar indicates 200 µm.
[FIG. 10] Phase-contrast microscope images and fluorescence microscope images observing localization of the compound (P1). The drawing in the left column is an estimated prediction schematic diagram.
[FIG. 11] Phase-contrast microscope images showing changes in droplets due to a light irradiation operation.
[FIG. 12] A schematic diagram showing changes in the state of a droplet dispersion liquid, which are estimated to occur due to the light irradiation operation of FIG. 11.
[FIG. 13] A transmission electron microscope image of a dispersion liquid in which the compound (P1) is in the trans form.
[FIG. 14] Viscosity measurement results for the trans form of the compound (P1).
[FIG. 15] Infrared absorption analysis results for the trans form of the compound (P1).
[FIG. 16] Gel-sol conversion of the compound (P1) by light irradiation.
[FIG. 17] Fusion of droplets due to the light irradiation of droplets produced in coexistence with a compound (P2) synthesized in Example 2. The scale bar indicates 100 µm.
[FIG. 18] Ultraviolet-visible light analysis results for a compound (P3) synthesized in Example 3.
[FIG. 19] Infrared absorption spectroscopy analysis results for the compound (P3).
[FIG. 20] Transmission electron microscope images of a dispersion liquid of the compound (P3).
[FIG. 21] Viscosity measurement results for the compound (P3).
[FIG. 22] Gel-sol conversion of the compound (P3) due to light irradiation.

### DESCRIPTION OF EMBODIMENTS

The term "comprise" means that constituent elements other than the target constituent elements may be included. The term "consist of" means that constituent elements other than the target constituent elements are not included. The term "consist essentially of" means that constituent elements other than the target constituent elements are not included in a manner whereby a special function is exhibited (such as a manner in which the effect of the invention is completely lost). In the present specification, the term "comprise" encompasses the meanings of "consist of" and "consist essentially of".

Peptides, proteins, nucleic acids, cells, and the like may be isolated. The term "isolated" means in a natural state or a state separated from other components. The term "isolated" may mean substantially not including other components. The phrase "substantially not including other components" means that the content of other components included in the isolated component is negligible. The content of other components included in the isolated component may be, for example, 10% by mass or less, 5% by mass or less, 4% by mass or less, 3% by mass or less, 2% by mass or less, 1% by mass or less, 0.5% by mass or less, or 0.1% by mass or less. The peptides described in the present specification may be isolated peptides, isolated proteins, isolated nucleic acids, and isolated cells.

The term "peptide" refers to a polymer of amino acids bonded by amide bonds.

The term "peptide structure" refers to a structure in which amino acids are bonded by amide bonds to form a polymer. A compound including a peptide structure may be a peptide, or a compound including a chemical structure other than a peptide. When referring to a specific peptide structure, the specific peptide structure may be a partial structure included in a single peptide molecule.

The term "amino acid" refers to a compound including an amino group and a carboxy group. An amino acid may be in an L-form or a D-form. An amino acid may be a natural amino acid or a non-natural amino acid. An amino acid may be an α-amino acid, a β-amino acid, or a γ-amino acid. An α-amino acid is represented by General Formula (α). A β-amino acid is represented by General Formula (β). A γ-amino acid is represented by General Formula (γ). The amino acid is preferably an α-amino acid.

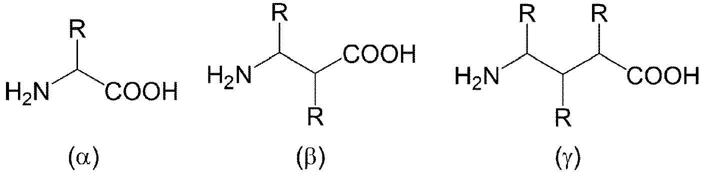

The side chain in an amino acid is a group that does not contribute to an amide bond when a peptide is formed. Specifically, the group represented by R in Formula (α)*,* (β), or (γ) is the side chain (amino acid side chain).

The term "alkyl group" encompasses linear, branched, and cyclic monovalent saturated hydrocarbon groups, unless otherwise specified. The same also applies to the alkyl group in an alkoxy group. The term "alkylene group" encompasses linear, branched, and cyclic divalent saturated hydrocarbon groups, unless otherwise specified.

The term "halogenated alkyl group" is a group in which some or all of the hydrogen atoms of an alkyl group are replaced with halogen atoms. Examples of the halogen atoms include fluorine atoms, chlorine atoms, bromine atoms, and iodine atoms. The same also applies to halogenated alkoxy groups.

Description that includes the phrase "may have a substituent" includes both a case where a hydrogen atom (-H) is replaced with a monovalent group and a case where a methylene group (-CH₂-) is replaced with a divalent group.

For a group for which the definition includes "may have a substituent", when referring to the number of carbon atoms, the carbon atoms of the substituent are usually not included.

In the present specification and the claims, depending on the structure represented by the chemical formula, asymmetric carbons may be present and enantiomers or diastereomers may be present. In such cases, these isomers are represented by a single chemical formula. These isomers may be used alone or as a mixture.

### <Compound (P)>

The first aspect of the present disclosure is a compound (also referred to below as "compound (P)") that includes a peptide structure (also referred to below as "peptide structure (p)") represented by General Formula (p1) or (p2).

XaXb(XaXc)ₙXaXdXa... (p1)

XaXd(XaXc)ₙXaXbXa... (p2)

[In the formulas, Xa represents an amino acid residue having a hydrophilic side chain; Xb represents an amino acid residue having a side chain represented by General Formula (bl); Xc and Xd each independently represent an amino acid residue or a glycine residue having a hydrophobic side chain; n represents an integer of 1 to 3. The plurality of Xa's may each be identical or different. When n is 2 or 3, the plurality of Xc's may each be identical or different.]

*-Ar²-N=N-Ar¹ (b1)

[In the formula, Ar¹ represents an aryl group which may have a substituent or a heteroaryl group which may have a substituent; and Ar² represents an arylene group which may have a substituent or a heteroarylene group which may have a substituent. * represents a bond.]

In Formula (p1) or (p2), Xa represents an amino acid residue having a hydrophilic side chain. The hydrophilic side chain is an amino acid side chain having a hydrophilic group. Xa is preferably an amino acid residue derived from an α-amino acid. Specific examples of Xa include arginine residue, asparagine residue, aspartic acid residue, glutamic acid residue, glutamine residue, lysine residue, serine residue, threonine residue, cysteine residue, histidine residue, ornithine residue, and the like. In Formula (p1) or (p2), the plurality of Xa's may each be identical or different.

In Formula (p1) or (p2), Xb represents an amino acid residue having a side chain represented by Formula (b1) (also referred to below as "side chain (b1)"). In Formula (b1), * is a bond bonded to a carbon atom constituting the main chain of an amino acid polymer. Xb is preferably an amino acid residue derived from an α-amino acid. When Xb is an amino acid residue derived from an α-amino acid, the α-amino acid from which Xb is derived is represented by Formula (Xb-1). 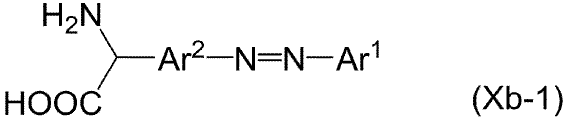 [In the formula, Ar¹ and Ar² are the same as Ar¹ and Ar² in Formula (b1).]

In Formula (b1), Ar¹ represents an aryl group which may have a substituent or a heteroaryl group which may have a substituent. The aryl group in Ar¹ preferably has 6 to 18 carbon atoms, more preferably has 6 to 15 carbon atoms, and even more preferably has 6 to 12 carbon atoms. The aryl group may be a monocyclic group or a polycyclic group. The number of aromatic hydrocarbon rings included in the aryl group is preferably 1 to 3, more preferably 1 or 2, and even more preferably 1. Specific examples of the aryl group include a phenyl group, a naphthyl group, an anthrenyl group, a phenanthrenyl group, and the like.

The heteroaryl group in Ar¹ preferably has 1 to 18 carbon atoms, more preferably 2 to 15 carbon atoms, and even more preferably 3 to 12 carbon atoms. For the heteroaryl group, examples of a hetero atom included in an aromatic heterocycle include an oxygen atom, a nitrogen atom, or a sulfur atom. The heteroaryl group may be a monocyclic group or a polycyclic group. The heteroaryl group may be a monocyclic group of an aromatic heterocycle, a condensed cyclic group of an aromatic hydrocarbon ring and an aromatic heterocycle, or a condensed ring of aromatic heterocycles. The total number of aromatic heterocycles and aromatic hydrocarbon rings included in the heteroaryl group is preferably 1 to 3, more preferably 1 or 2, and even more preferably 1.

Examples of aromatic heterocycles included in heteroaryl groups include nitrogen-containing aromatic heterocycles (pyrrole rings, pyridine rings, azepine rings, azocine rings, azonine rings, imidazole rings, pyrazole rings, imidazoline rings, triazole rings, tetrazole rings, pyridazine rings, pyrimidine rings, pyrazine rings, and the like); condensed rings of nitrogen-containing aromatic heterocycles and benzene rings (indole rings, isoindole rings, benzimidazole rings, purine rings, benzotriazole rings, quinoline rings, isoquinoline rings, quinazoline rings, quinoxaline rings, cinnoline rings, and the like); sulfur-containing aromatic heterocycles (thiol rings, thiepine rings, thionine rings, and the like); aromatic heterocycles including nitrogen atoms and sulfur atoms (thiazole rings, thiazine rings, and the like); oxygen-containing aromatic heterocycles (oxole rings, oxepin rings, oxonine rings, and the like); aromatic heterocycles including nitrogen atoms and oxygen atoms (oxazole rings), and the like, without being limited thereto.

The aryl group or heteroaryl group in Ar¹ may or may not have a substituent. The substituent is not particularly limited, but is preferably a hydrophobic group. Examples of the substituent include an alkyl group, a halogenated alkyl group, an alkoxy group, a halogenated alkoxy group, a halogen atom, and the like. As the substituent, the alkyl group and alkoxy group preferably have 1 to 12 carbon atoms, more preferably have 1 to 10 carbon atoms, even more preferably have 1 to 6 carbon atoms, and particularly preferably have 1 to 3 carbon atoms. Examples of the halogen atom in the halogenated alkyl group and halogenated alkoxy group and the halogen atom as the substituent include a fluorine atom, a bromine atom, and an iodine atom, and a fluorine atom is preferable.

Ar¹ is preferably an aryl group which may have a substituent, more preferably a phenyl group or naphthyl group which may have a substituent, even more preferably a phenyl group which may have a substituent, and particularly preferably a perfluorophenyl group (pentafluorophenyl group) or a phenyl group.

In Formula (b1), Ar² represents an arylene group which may have a substituent or a heteroarylene group which may have a substituent. The arylene group in Ar² preferably has 6 to 18 carbon atoms, more preferably has 6 to 15 carbon atoms, and even more preferably has 6 to 12 carbon atoms. The arylene group may be a monocyclic group or a polycyclic group. The number of aromatic hydrocarbon rings included in the arylene group is preferably 1 to 3, more preferably 1 or 2, and even more preferably 1. Specific examples of the arylene group include a phenylene group, a naphthylene group, an anthracenediyl group, a phenanthrenediyl group, and the like.

The heteroarylene group in Ar² preferably has 1 to 18 carbon atoms, more preferably has 2 to 15 carbon atoms, and even more preferably has 3 to 12 carbon atoms. Examples of the heteroarylene group include, as the hetero atom included in the aromatic heterocycle, an oxygen atom, a nitrogen atom, and a sulfur atom. The heteroarylene group may be a monocyclic group or a polycyclic group. The heteroarylene group may be a monocyclic group of an aromatic heterocycle, a condensed cyclic group of an aromatic hydrocarbon ring and an aromatic heterocycle, or a condensed ring of aromatic heterocycles. The total number of aromatic heterocycles and aromatic hydrocarbon rings included in the heteroaryl group is preferably 1 to 3, more preferably 1 or 2, and even more preferably 1.

Examples of the aromatic heterocycle included in the heteroarylene group are the same as for the heteroaryl group in Ar¹ described above.

The arylene group or heteroarylene group in Ar² may or may not have a substituent. The substituent is not particularly limited, but is preferably a hydrophobic group. Examples of the substituent are the same as for the substituent in Ar¹ described above.

Ar² is preferably an arylene group which may have a substituent, more preferably a phenylene group or naphthylene group which may have a substituent, and even more preferably a phenylene group.

The side chain (b1) is preferably a side chain represented by General Formula (b1-1). 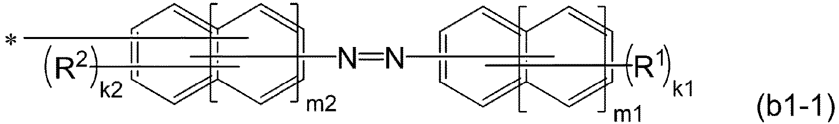 [In the formula, m1 and m2 each independently represent an integer of 0 to 3; R¹ and R² each independently represent a substituent; k1 and k2 each independently represent an integer of 0 or more as allowed by an atomic valence. When k1 is an integer of 2 or more, two or more R¹'s may each be identical or different. When k2 is an integer of 2 or more, the two or more R²'s may each be identical or different. * represents a bond.]

In Formula (b1-1), m1 and m2 each independently represent an integer of 0 to 3. m1 and 2 are preferably 0 to 2, more preferably 0 or 1, and even more preferably 0.

In Formula (b1-1), R¹ and R² each independently represent a substituent. The substituent is not particularly limited, but is preferably a hydrophobic group. Specific examples of the substituent include the same examples as for the substituent in Ar¹ in Formula (b1). R¹ and R² are preferably halogen atoms and more preferably fluorine atoms.

When m1 is 0, k1 is an integer of 0 to 5. When m1 is 1, k1 is an integer of 0 to 7. When m1 is 2, k1 is an integer of 0 to 9. When m1 is 3, k1 is an integer of 0 to 11. m1 is preferably 0 and k1 is preferably 0 or 5. When m2 is 0, k2 is an integer of 0 to 4. When m2 is 1, k2 is an integer of 0 to 6. When m2 is 2, k2 is an integer of 0 to 8. When m2 is 3, k2 is an integer of 0 to 10. m2 is preferably 0 and k1 is preferably 0.

Specific examples of the side chain (b1) are shown below, without being limited thereto. In the following formula, * represents a bond bonded to a carbon atom constituting the main chain of the amino acid polymer. The following specific examples show the trans form, but the cis form is also acceptable.

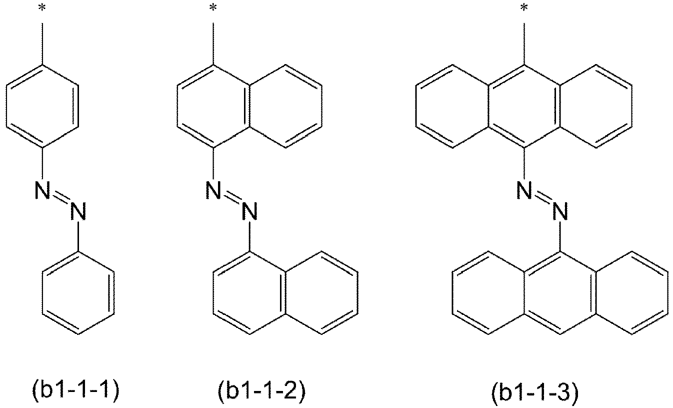

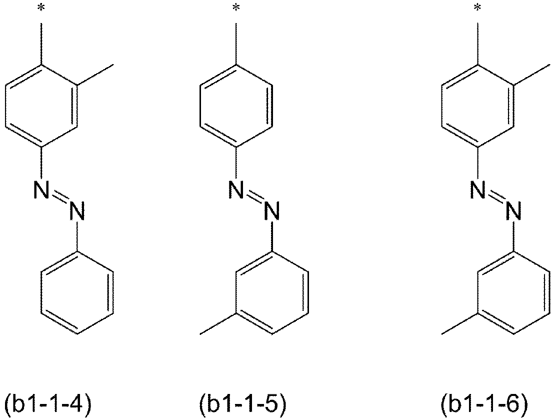

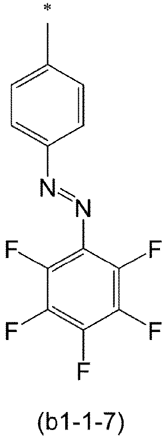

In Formula (p1) or (p2), Xc and Xd represent an amino acid residue or a glycine residue having a hydrophobic side chain. As the amino acid residue having a hydrophobic side chain, an amino acid residue derived from an α-amino acid is preferable. Specific examples of amino acid residues having a hydrophobic side chain include an alanine residue, a valine residue, a leucine residue, an isoleucine residue, a methionine residue, a proline residue, a phenylalanine residue, a tryptophan residue, and a tyrosine residue.

From the viewpoint of the solubility of the compound (P) in water, Xc is preferably an alanine residue or a glycine residue. When n in Formula (p1) or (p2) is 2 or 3, the plurality of Xc's may each be identical or different.

Xd is preferably an amino acid residue having a hydrophobic side chain having 3 or more carbon atoms. The hydrophobic side chain having 3 or more carbon atoms preferably has 3 to 12 carbon atoms, more preferably 3 to 10 carbon atoms, and even more preferably 3 to 8 carbon atoms. Specific examples of Xd include valine residue, leucine residue, isoleucine residue, methionine residue, proline residue, phenylalanine residue, tryptophan residue, tyrosine residue, and the like.

In Formula (p1) or (p2), n represents an integer of 1 to 3, preferably 1 or 2, and more preferably 1.

The peptide structure (p) is preferably a peptide structure represented by General Formula (p1-1) or (p2-1). 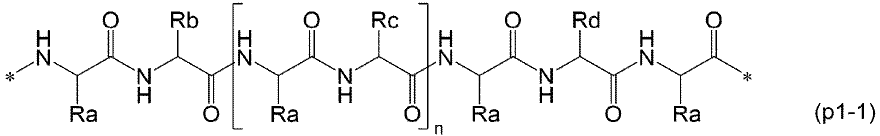 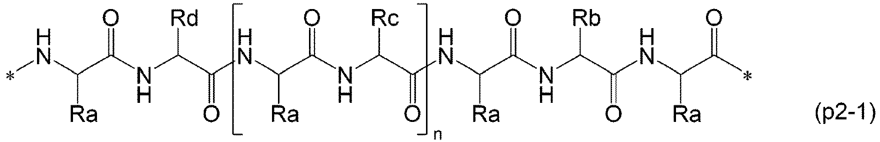 [In the formula, Ra represents a hydrophilic side chain; Rb represents a side chain represented by General Formula (b1); Rc and Rd each independently represent a hydrophobic side chain or a hydrogen atom; and n represents an integer of 1 to 3. A plurality of Ra's may each be identical or different. When n is 2 or 3, the plurality of Rc's may each be identical or different. * represents a bond.]

In Formula (p1-1) or (p2-1), Ra represents a hydrophilic side chain. Examples of the hydrophilic side chain include an aliphatic hydrocarbon group having a hydrophilic group. Examples of the hydrophilic group include an amino group (preferably a primary amino group), a carboxy group, a hydroxyl group (preferably an alcoholic hydroxyl group), a thiol group, and the like. The aliphatic hydrocarbon group may be a saturated aliphatic hydrocarbon group or an unsaturated aliphatic hydrocarbon group, but is preferably a saturated aliphatic hydrocarbon group. The aliphatic hydrocarbon group may be linear, branched, or cyclic, but is preferably linear or branched, and is preferably linear. The aliphatic hydrocarbon group preferably has 1 to 6 carbon atoms, more preferably 1 to 4 carbon atoms, and even more preferably 1 to 3 carbon atoms.

Examples of the hydrophilic side chain in Ra include the side chain of a hydrophilic amino acid. Specific examples of hydrophilic amino acids include arginine, asparagine, aspartic acid, glutamine, glutamic acid, lysine, serine, threonine, cysteine, histidine, ornithine, and the like. Specific examples of Ra are shown below, without being limited thereto. In Formula (p1-1) or (p2-1), a plurality of Ra's may each be identical or different. In the following formula, * represents a bond bonded to a carbon atom constituting the main chain of the amino acid polymer in Formula (p1-1) or (p2-1). The reference symbol in parentheses represents the derivation source amino acid.

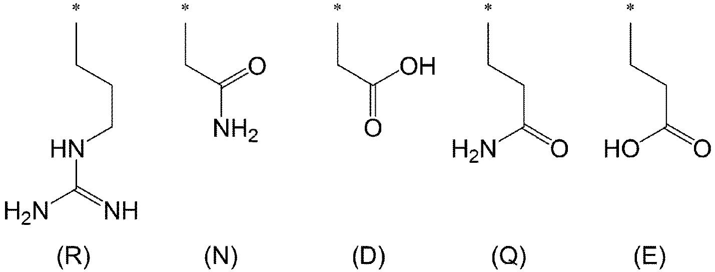

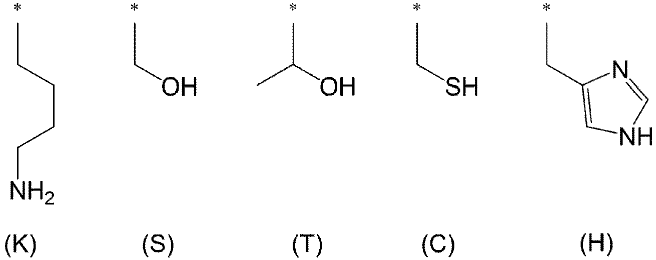

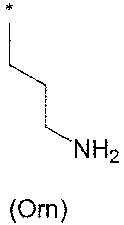

In Formula (p1-1) or (p2-1), Rb represents a side chain represented by Formula (b1). Rb is preferably a side chain represented by Formula (b1-1) and more preferably a side chain represented by any of Formulas (b1-1-1) to (b1-1-7).

In Formula (p1-1) or (p2-1), Rc and Rd represent a hydrophobic side chain or a hydrogen atom. Examples of the hydrophobic side chain include a hydrocarbon group having one or more carbon atoms, which may have a substituent. The hydrocarbon group preferably has 1 to 12 carbon atoms, more preferably 1 to 10 carbon atoms, and even more preferably 1 to 8 carbon atoms. The hydrocarbon group may be an aromatic hydrocarbon group or an aliphatic hydrocarbon group. The aliphatic hydrocarbon group may be a saturated aliphatic hydrocarbon group or an unsaturated aliphatic hydrocarbon group, but is preferably a saturated aliphatic hydrocarbon group. The aliphatic hydrocarbon group may be linear, branched, or cyclic. The hydrocarbon group may have a substituent as long as the substituent is not hydrophilic. The hydrophobic side chain in Rc and Rd may be a side chain of a hydrophobic amino acid. Examples of the hydrophobic amino acid include the same examples as for Xc and Xd.

Rc is preferably a hydrogen atom or a methyl group from the viewpoint of the solubility of the compound (P) in water. When n in Formula (p1-1) or (p2-1) is 2 or 3, the plurality of Rc's may each be identical or different.

Rd is preferably a hydrophobic side chain having 3 or more carbon atoms. Examples of hydrophobic side chains having 3 or more carbon atoms include hydrocarbon groups having 3 or more carbon atoms which may have a substituent. The hydrocarbon group preferably has 3 to 12 carbon atoms, more preferably has 3 to 10 carbon atoms, and even more preferably has 3 to 8 carbon atoms. The hydrocarbon group may be an aromatic hydrocarbon group or an aliphatic hydrocarbon group. The aliphatic hydrocarbon group may be a saturated aliphatic hydrocarbon group or an unsaturated aliphatic hydrocarbon group, but is preferably a saturated aliphatic hydrocarbon group. The aliphatic hydrocarbon group may be linear, branched, or cyclic. The hydrocarbon group may have a substituent as long as the substituent is not hydrophilic. Examples of the substituent that the hydrocarbon group in Rd may have include a secondary amino group, a tertiary amino group, a sulfide group, a phenolic hydroxyl group, and the like.

Examples of hydrophobic side chains having 3 or more carbon atoms include side chains of hydrophobic amino acids having 3 or more carbon atoms. Specific examples of such hydrophobic amino acids include valine, leucine, isoleucine, methionine, proline, phenylalanine, tryptophan, tyrosine, and the like. Specific examples of Rd are shown below, without being limited thereto. When n in Formula (p1-1) or (p2-1) is 2 or 3, the plurality of Rd's may each be identical or different. In the following formula, * represents a bond bonded to a carbon atom constituting the main chain of the amino acid polymer in Formula (p1-1) or (p2-1). The symbol in parentheses represents the derivation source amino acid.

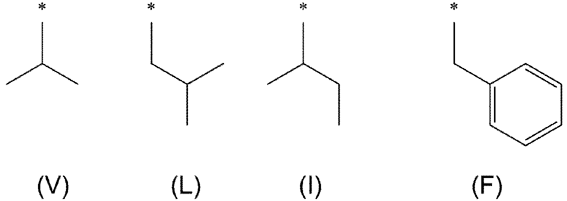

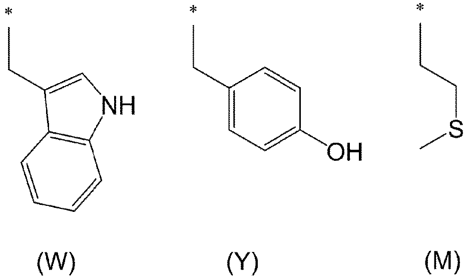

When Rd is a proline side chain, Rd has the following structure together with the carbon atom to which Rd is bonded and the NH group adjacent to this carbon atom in Formula (p1-1) or Formula (p2-1).

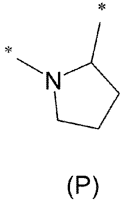

In Formula (p1-1) or (p2-1), n represents an integer of 1 to 3, preferably 1 or 2, and more preferably 1.

In Formula (p1-1) or (p2-1), examples of combinations of Ra, Rb, Rc, and Rd include combinations in which Ra is a side chain of an amino acid selected from the group consisting of arginine, asparagine, aspartic acid, glutamine, glutamic acid, lysine, serine, threonine, cysteine, histidine, and ornithine; Rb is a side chain represented by Formula (b1-1); Rc is a hydrogen atom or a methyl group; and Rd is a side chain of an amino acid selected from the group consisting of valine, leucine, isoleucine, methionine, proline, phenylalanine, tryptophan, and tyrosine. In the above-described combinations, n in Formula (p1-1) or (p2-1) is preferably 1 or 2 and more preferably 1.

In Formula (p1-1) or (p2-1), the combination of Ra, Rb, Rc, and Rd is preferably a combination in which Ra is a side chain of arginine or aspartic acid; Rb is a side chain represented by Formula (b1-1); Rc is a hydrogen atom or a methyl group; and Rd is a side chain of methionine. In the above-described combination, n in Formula (p1-1) or (p2-1) is preferably 1 or 2 and more preferably 1.

Specific examples of the peptide structure (p) are shown below, without being limited thereto. In the following specific examples, the side chain (b1) is shown in the trans form, but the side chain (b1) may be in the cis form. * is a bond bonded to an adjacent atom.

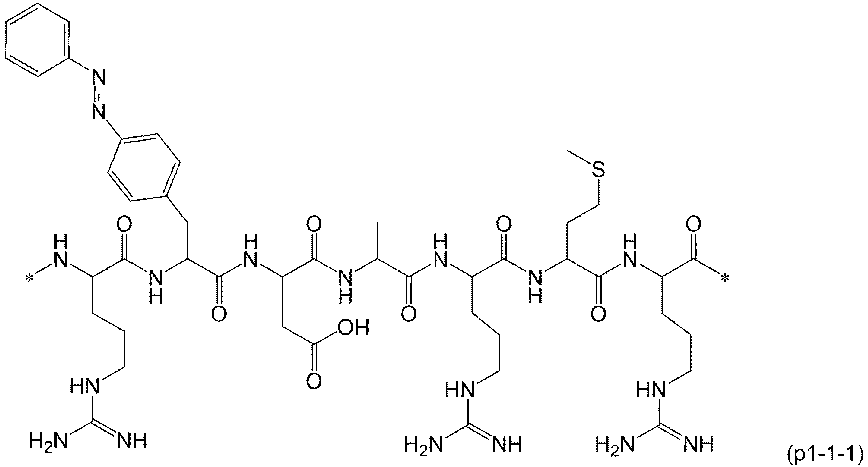

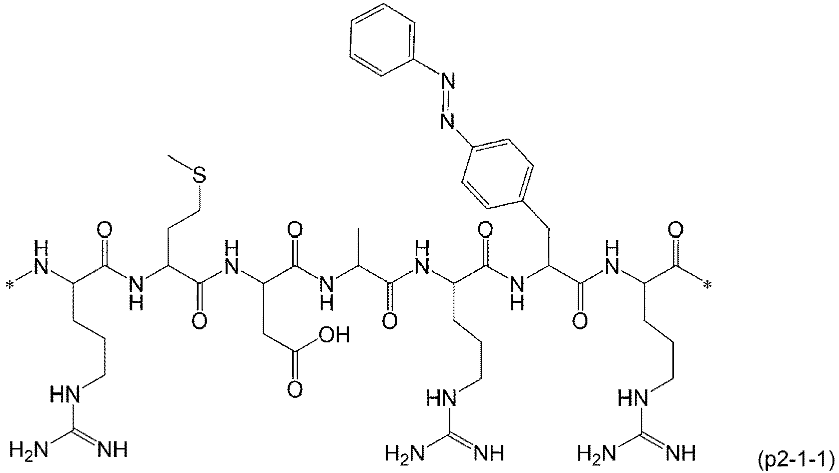

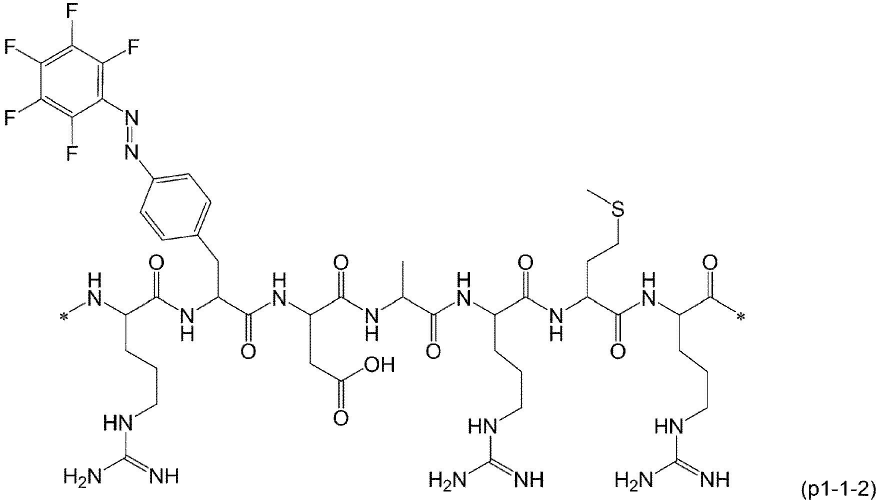

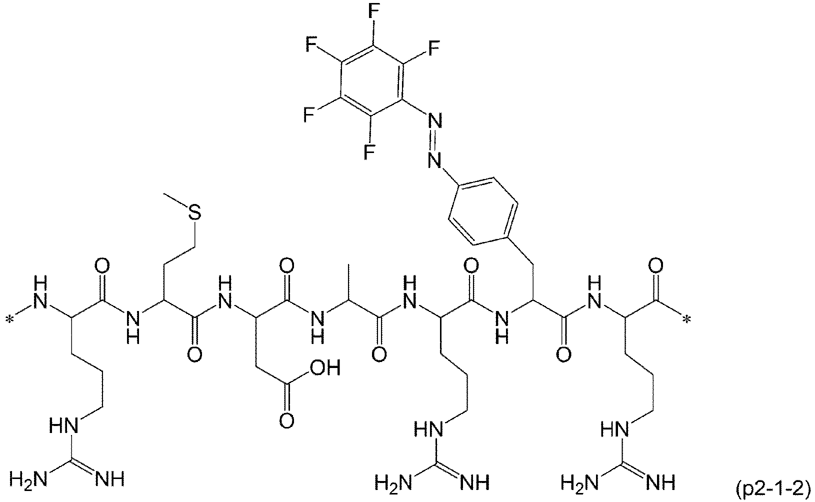

In the compound (P), structures other than the peptide structure (p) are not particularly limited. The compound (P) may be, for example, a peptide including the peptide structure (p). In this case, the number of amino acid residues of the compound (P) is not particularly limited and examples thereof include 40 or less, 30 or less, 25 or less, 20 or less, 15 or less, or 10 or less. The number of amino acid residues includes the number of amino acid residues of the peptide structure (p). When the compound (P) is a peptide including the peptide structure (p), the type of the amino acid residues in the region other than the peptide structure (p) is not particularly limited. The portion of the compound (P) other than the peptide structure (p) does not have to be a peptide. For example, an organic group having 1 to 30 carbon atoms may be bonded to either or both of the two ends of the peptide structure (p). The compound (P) may be a compound represented by General Formula (p1-1') or (p2-1'). 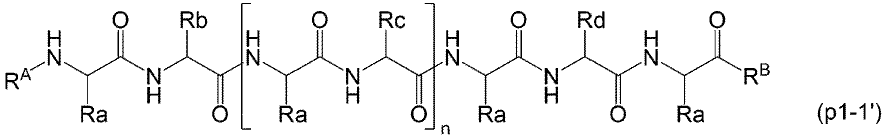 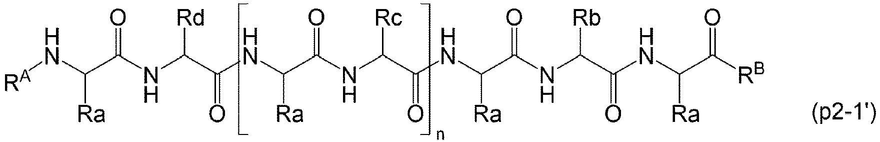 [In the formulas, R^{A} and R^{B} each independently represent a monovalent group. Ra, Rb, Rc, Rd, and n are the same as Ra, Rb, Rc, Rd, and n in Formula (p1-1) or (p2-1).]

The monovalent groups in R^{A} and R^{B} in General Formula (p1-1') or (p2-1') are not particularly limited. Examples of the monovalent groups in R^{A} and R^{B} include an acetyl group, an amino group, a hydroxy group, a hydrogen atom, a halogen atom, a monovalent organic group, and the like. Examples of the monovalent organic group include a peptide consisting of 1 to 20 amino acid residues and monovalent organic groups other than peptides. Examples of the monovalent organic groups other than peptides include a hydrocarbon group which may have a substituent. Specific examples include alkyl groups, polyoxyethylene groups, polyoxypropylene groups, phenyl groups, and the like, without being limited thereto.

Specific examples of the compound (P) are shown below, without being limited thereto. In the following specific examples, the side chain (b1) is shown in the trans form, but the side chain (b1) may be in the cis form.

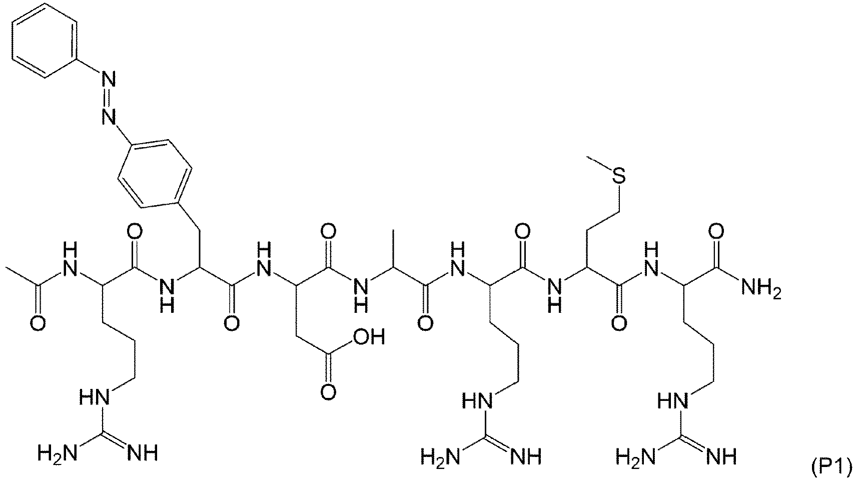

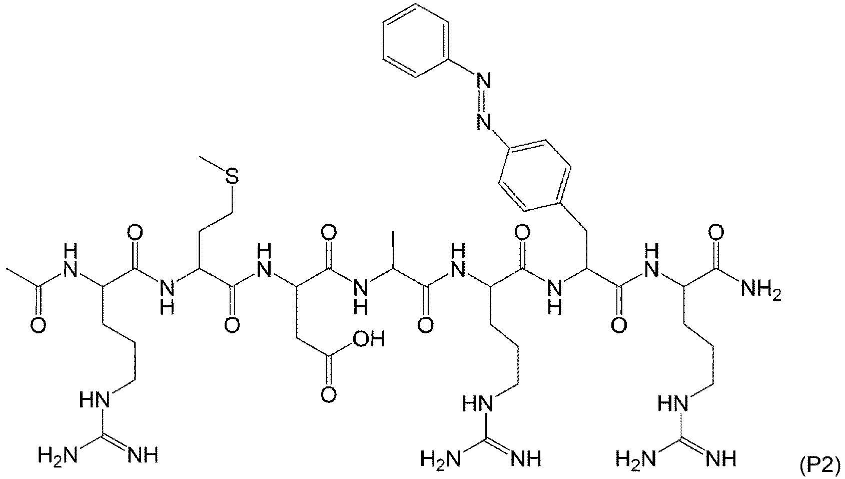

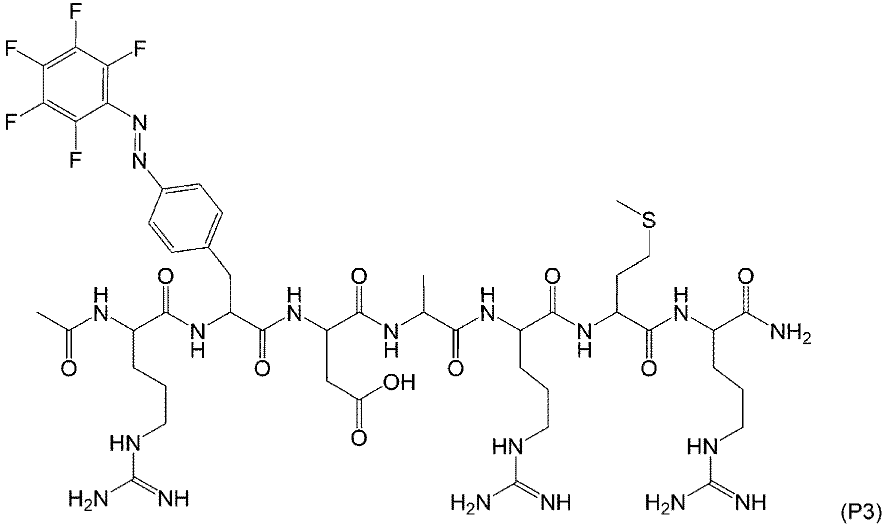

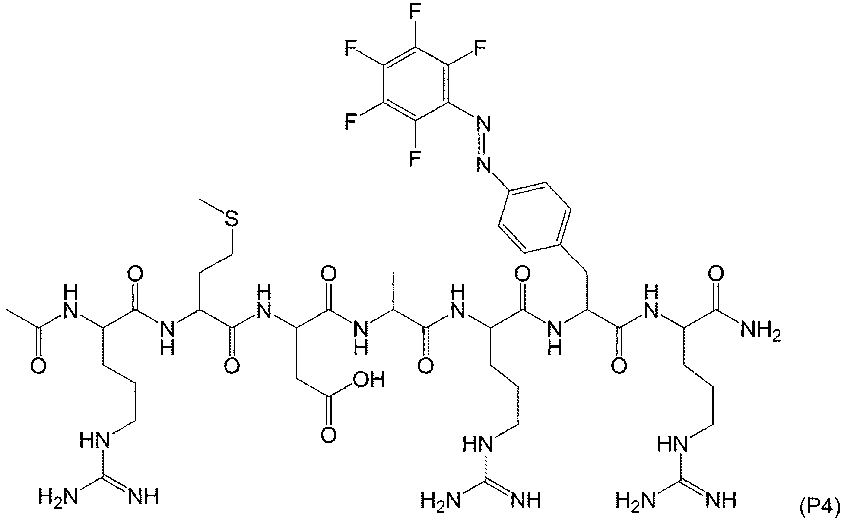

It is possible to produce the compound (P) by a known peptide synthesis method such as solid-phase peptide synthesis. It is possible to use commercially available amino acids as the raw material for the compound (P).

It is possible to use the compound (P) to produce liquid-liquid phase-separated droplets. When liquid-liquid phase-separated droplets are formed from an aqueous solution including a polysaccharide and a polyether, the presence of the compound (P) in the aqueous solution makes it possible to stably maintain the droplets for a long period of time. Alternatively, it is possible to stably maintain the droplets for a long period of time by forming liquid-liquid phase-separated droplets from an aqueous solution including a polysaccharide and a polyether and then adding the compound (P) to the aqueous solution including the liquid-liquid phase-separated droplets.

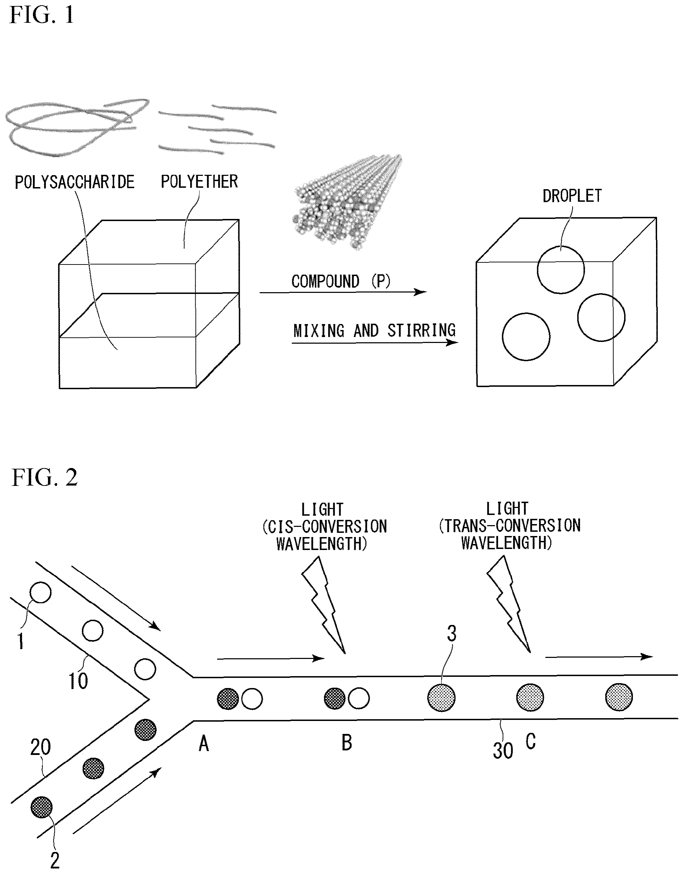

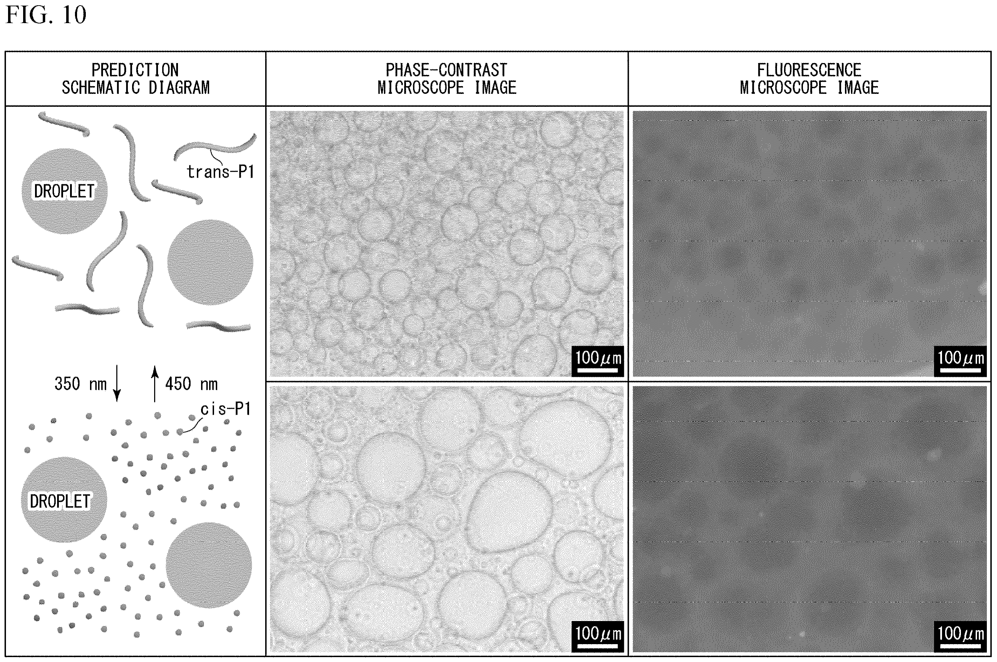

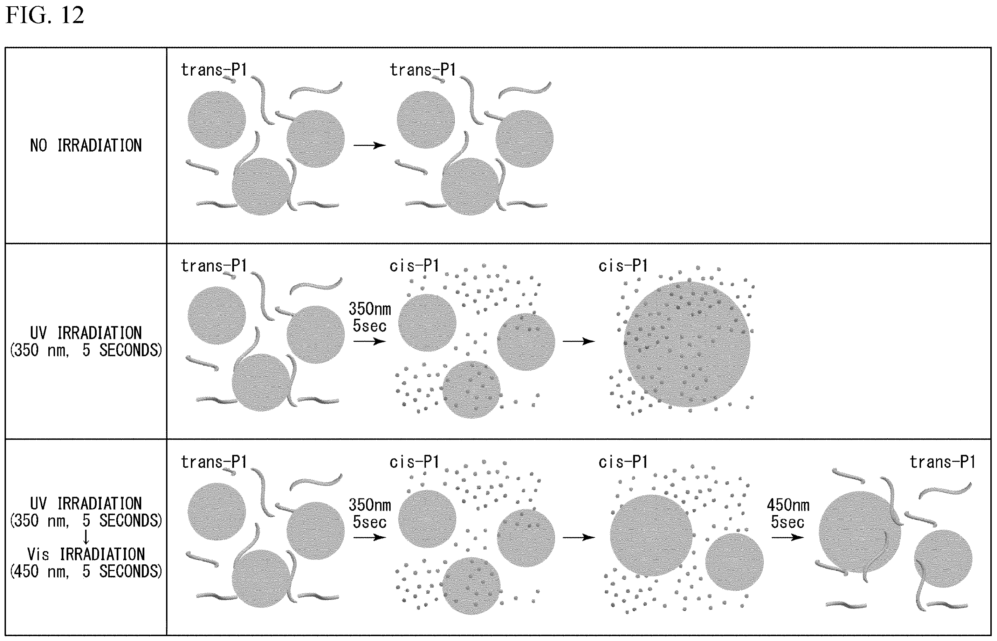

FIG. 1 is a schematic diagram showing an example of a method for producing liquid-liquid phase-separated droplets using the compound (P). When an aqueous solution including a polysaccharide and a polyether is mixed and stirred, extremely small droplets including a high concentration of polysaccharide are formed by liquid-liquid phase separation. However, the formed droplets are unstable and the droplets fuse together over time, changing to macro-scale phase separation. On the other hand, when the compound (P) is added to the aqueous solution, mixed, and stirred to form droplets, the droplets are stabilized and it is possible to maintain the extremely small droplets for a long time. This is estimated to be because the compound (P) is localized at the interface of the droplets, stabilizing the droplets. As shown in FIG. 10 and FIG. 12, when the compound (P) is in the trans form, the compound (P) is considered to be present in a fibrous state outside the droplets. The presence of the compound (P) in a fibrous state is considered to cause the solution around the droplets to undergo gelation, contributing to the stabilization of the droplets.

Furthermore, it is possible to fuse together droplets formed in the presence of the compound (P) or droplets coexisting with the compound (P) by light irradiation. The fusion of the droplets occurs position-specifically where irradiated with light. Therefore, the light irradiation makes it possible to fuse droplets at a desired position and at a desired timing. This is considered to be a phenomenon that occurs because the side chain (b1) in the compound (P) is photoisomerized by light irradiation. For example, when the side chain (b1) is a side chain represented by Formula (b1-1-1), the side chain (b1) in the compound (P) is photoisomerized from the trans form to the cis form by irradiating the compound (P) with 200 nm to 400 nm light (preferably 300 nm to 380 nm, for example, 350 nm). In addition, for example, when the side chain (b1) is a side chain represented by Formula (b1-1-7), the side chain (b1) in the compound (P) is photoisomerized from the trans form to the cis form by irradiating the compound (P) with 400 nm to 700 nm light (preferably 500 nm to 600 nm, for example, 540 nm). It is considered that the fusing together of the droplets occurs alongside this photoisomerization. As shown in FIG. 10 and FIG. 12, when the compound (P) is in the cis form, it is considered that the fiber shape is broken and the compound (P) is dispersed outside the droplets. It is considered that the fiber shape of the compound (P) decomposes, causing the solution around the droplets to undergo solation and causing the fusing together of the droplets to advance.

The photoisomerization of the compound (P) is reversible. For example, when the side chain (b1) is a side chain represented by Formula (b1-1-1) or (b1-1-7), the side chain (b1) in the compound (P) is converted from the cis form to the trans form by irradiating the compound (P) with 410 nm to 600 nm light (preferably 420 nm to 500 nm, for example, 450 nm). The wavelength at which photoisomerization of the compound (P) occurs may vary depending on the structure of the compound (P) (in particular, the structure of the side chain (b1)). It is possible to confirm the wavelength at which photoisomerization of the compound (P) occurs by measuring the absorption spectrum of the compound (P) by, for example, ultraviolet-visible light spectroscopy, circular dichroism spectrum analysis, or the like. For example, an aqueous solution in which the compound (P) is dissolved is irradiated with light and then the absorption spectrum of the aqueous solution is measured. It is possible to set an irradiation light wavelength at which the absorption peak near 320 nm decreases and the absorption peak near 430 nm increases as the irradiation time increases as the wavelength at which the compound (P) undergoes cis-conversion. It is possible to set an irradiation light wavelength at which the absorption peak near 320 nm increases and the absorption peak near 430 nm decreases as the irradiation time increases as the wavelength at which the compound (P) undergoes trans-conversion.

In the present specification, the cis-conversion of the compound (P) means that the side chain (b1) in the compound (P) is converted from a trans type to a cis type. The trans-conversion of the compound (P) means that the side chain (b1) in the compound (P) is converted from a cis type to a trans type. The compound (P) in which the side chain (b1) is in the trans form may be referred to as the "trans form of the compound (P)". The compound (P) in which the side chain (b1) is in the cis form may be referred to as the "cis form of the compound (P)".

### <Gelling Agent>

The second aspect of the present disclosure is a gelling agent. The gelling agent of the present embodiment includes the compound (P).

It is possible to add the trans form of the compound (P) to an aqueous liquid to induce gelation of the aqueous liquid. Therefore, it is possible to use the compound (P) as a gelling agent. When a gel that underwent gelation as a result of adding the trans form of the compound (P) is irradiated with light having a wavelength that causes the compound (P) to undergo cis-conversion, the compound (P) in the gel undergoes cis-conversion. As a result, the gel undergoes solation.

On the other hand, an aqueous liquid including the cis form of the compound (P) is a sol-like liquid, but when irradiated with light having a wavelength that trans-converts the compound (P), the compound (P) undergoes trans-conversion. As a result, the aqueous liquid undergoes gelation.

As described above, it is possible to control the gelation of an aqueous liquid including the compound (P) by irradiation with light. Accordingly, it is possible to use the compound (P) as a gelation control agent for controlling a gel-sol transition.

The aqueous liquid to which it is possible to apply the gelling agent of the present embodiment is a liquid including water as a solvent. The aqueous liquid may be water, but preferably includes a solute from the viewpoint of the solubility of the compound (P). The content of the water in the aqueous liquid is preferably 60% by mass or more and less than 100% by mass with respect to the total mass of the aqueous composition, more preferably 70% by mass or more and less than 100% by mass, and even more preferably 80% by mass or more and less than 100% by mass. The aqueous liquid may include a solvent other than water, but preferably does not include any solvent.

The solute in the aqueous liquid is not particularly limited and examples thereof include sugars (monosaccharides, disaccharides, oligosaccharides, polysaccharides, and the like), polyethers (polyethylene glycol, polypropylene glycol, and the like), salts (inorganic salts, organic salts), inorganic acids, organic acids, inorganic bases, organic bases, and the like. Specific examples of aqueous liquids include various buffer solutions such as phosphate buffered saline, acetate buffer solutions, phosphate buffer solutions, citrate buffer solutions, citrate phosphate buffer solutions, borate buffer solutions, tartrate buffer solutions, Tris buffer solutions, and HEPES buffer solutions; and various liquid media such as Dulbecco's modified Eagle's medium (D-MEM) and MEMα medium.

The aqueous liquid preferably has a pH of 4 to 10, more preferably a pH of 5 to 9, even more preferably a pH of 6 to 9, and particularly preferably a pH of 7 to 9. When the pH of the aqueous liquid is in the above-described range, it is easy to control the gel-sol transition caused by the photoisomerization of the compound (P).

It is possible to add the compound (P) to the aqueous liquid such that the final concentration in the aqueous liquid is, for example, 0.2% by mass or more and 8% by mass or less, 0.4% by mass or more and 6% by mass or less, 0.5% by mass or more and 5% by mass or less, or 0.8% by mass or more and 3% by mass or less.

The gelling agent of the present embodiment may consist of only the compound (P), or may include other components in addition to the compound (P). The other components are not particularly limited as long as the gelation action of the compound (P) is not inhibited.

According to the gelling agent of the present embodiment, it is possible to control the sol-gel conversion by irradiation with light.

### <Droplet Production Kit>

The third aspect of the present disclosure is a droplet production kit. The droplet production kit of the present embodiment includes the compound (P), a polysaccharide, and a polyether.

### (Compound (P))

The compound (P) is the same as described above. The compound (P) may be a compound in which the side chain (b1) is either in the trans form or the cis form, but is preferably a compound in which the side chain is in the trans form.

### (Polysaccharide)

The polysaccharide is not particularly limited. Examples of polysaccharides include dextran, dextrin, amylose, amylopectin, glycogen, cellulose, agarose, carrageenan, heparin, alginic acid, hyaluronic acid, pectin, glucomannan, tamarind seed gum, guar gum, locust bean gum, gum arabic, karaya gum, xanthan gum, gellan gum, and the like, without being limited thereto. Polysaccharides containing glucose as a constituent sugar are preferable, dextran or dextrin are more preferable, and dextran is even more preferable.

The molecular weight of the polysaccharide is not particularly limited and examples thereof include 10,000 or more and 10,000,000 or less. The molecular weight of the polysaccharide may be 10,000 or more, 50,000 or more, 100,000 or more, 150,000 or more, 200,000 or more, 250,000 or more, 300,000 or more, 350,000 or more, 400,000 or more, 450,000 or more, 500,000 or more, or 550,000 or more. The upper limit value of the molecular weight of the polysaccharide is not particularly limited and may be, for example, 10,000,000 or less, 5,000,000 or less, 4,000,000 or less, 3,000,000 or less, 2,000,000 or less, 1,000,000 or less, 900,000 or less, 800,000 or less, 700,000 or less, 600,000 or less, or 550,000 or less. It is possible to combine the upper limit value and the lower limit value in any way. Examples of the molecular weight of the polysaccharide include 50,000 or more and 5,000,000 or less, 100,000 or more and 5,000,000 or less, 300,000 or more and 1,000,000 or less, and 400,000 or more and 800,000 or less.

The polysaccharide may be labeled using a fluorescent dye or the like. When liquid-liquid phase-separated droplets are formed, the polysaccharide is localized in the droplet. Therefore, by using polysaccharides labeled using different types of fluorescent dyes for each droplet production condition, it is possible to identify the droplet production conditions using the fluorescent dye as an indicator. The fluorescent dye is not particularly limited and it is possible to use any known fluorescent dye. To avoid droplet fusion, it is preferable to use a fluorescent dye having an excitation light wavelength and a fluorescent wavelength different from the wavelength at which the compound (P) undergoes cis-conversion. For example, when the compound (P) having a side chain represented by Formula (b1-1-1) is used, it is possible to use a fluorescent dye having an excitation light wavelength and a fluorescent wavelength of 490 nm or more. Examples of such fluorescent dyes include rhodamine, 5-FAM, 5-FITC, ATTO520, AlexaFlour555, Cy3Cy3, Cy5, Alexa568, Alexa647, and the like, without being limited thereto.

### (Polyether)

Polyethers are not particularly limited. Polyethers are compounds having a plurality of ether bonds in the molecule. Polyethers may be aliphatic polyethers or aromatic polyethers, and aliphatic polyethers are preferable. An example of an aliphatic polyether is polyalkylene glycol. Examples of polyalkylene glycols include polyethylene glycol, polypropylene glycol, polytetramethylene glycol, polyoxyethylene polyoxypropylene glycol, and the like. The polyether is preferably polyethylene glycol.

The molecular weight of the polyether is not particularly limited and examples thereof include 300 or more and 10,000,000 or less. The molecular weight of the polyether may be 500 or more, 1,000 or more, 2,000 or more, 3,000 or more, 5,000 or more, 10,000 or more, 15,000 or more, 20,000 or more, 25,000 or more, 30,000 or more, or 35,000 or more. The upper limit value of the molecular weight of the polyether is not particularly limited and may be, for example, 10,000,000 or less, 5,000,000 or less, 4,000,000 or less, 3,000,000 or less, 2,000,000 or less, 1,000,000 or less, 900,000 or less, 800,000 or less, 700,000 or less, 600,000 or less, 500,000 or less, 400,000 or less, 300,000 or less, 200,000 or less, 100,000 or less, 80,000 or less, 60,000 or less, 50,000 or less, 40,000 or less, or 35,000 or less. It is possible to combine the upper limit value and the lower limit value in any way. Examples of the molecular weight of the polyether include 500 or more and 5,000,000 or less, 1,000 or more and 3,000,000 or less, 5,000 or more and 1,000,000 or less, and 10,000 or more and 500,000 or less.

### (Other Configurations)

The kit of the present embodiment may include other configurations in addition to the above-described configuration. Other configurations include, for example, distilled water, buffers, microtubes, microchannel devices, instructions, and the like.

It is possible to use the kit of the present embodiment to produce liquid-liquid phase-separated droplets.

### <Method for Producing Droplets>

The fourth aspect of the present disclosure is a method for producing droplets. The method for producing droplets of the present embodiment includes forming droplets by stirring a solution including the compound (P), polysaccharide, and polyether (also referred to below as a "droplet formation step").

### (Droplet Formation Step)

It is possible to produce a solution including the compound (P), polysaccharide, and polyether (also referred to below as "mixed solution") by dissolving the compound (P), polysaccharide, and polyether in water or a buffer solution. As the compound (P), polysaccharide, and polyether, it is possible to use the same examples as described above. It is preferable to use the compound (P) in which the side chain (b1) is in the trans form. As the water, it is preferable to use water of a grade that is usually used in biochemical tests or the like, such as distilled water, ion-exchanged water, and ultrapure water. As the buffer solution, it is possible to use any buffer solution that is usually used in biochemical tests or the like, without any particular limitation. Examples of buffer solutions include phosphate buffer solutions, phosphate buffered saline, acetate buffer solutions, citrate buffer solutions, Tris buffer solutions, HEPES buffer solutions, and the like, without being limited thereto.

The method for producing the mixed solution is not particularly limited and the production may be carried out, for example, by producing, and then mixing, each of a solution including a polysaccharide, a solution including a polyether, and a solution including the compound (P). Alternatively, a mixed solution may be produced by producing a solution including a polysaccharide and a polyether and adding the compound (P) to the solution.

The concentration of each component in the mixed solution is not particularly limited. Examples of the concentration of the compound (P) in the mixed solution include 0.1 to 10% by mass with respect to the total mass of the mixed solution. The concentration of the compound (P) in the mixed solution may be, for example, 0.2% by mass or more with respect to the total mass of the mixed solution, 0.3% by mass or more, 0.4% by mass or more, 0.5% by mass or more, 0.6% by mass or more, 0.7% by mass or more, 0.8% by mass or more, 0.9% by mass or more, or 1.0% by mass or more. The upper limit value of the concentration of the compound (P) in the mixed solution is not particularly limited and may be, for example, 9% by mass or less with respect to the total mass of the mixed solution, 8% by mass or less, 7% by mass or less, 6% by mass or less, 5% by mass or less, 4% by mass or less, 3% by mass or less, 2% by mass or less, or 1% by mass or less. It is possible to combine the upper limit value and the lower limit value in any way. Examples of the concentration of the compound (P) in the mixed solution include 0.2% by mass or more and 8% by mass or less, 0.4% by mass or more and 6% by mass or less, 0.5% by mass or more and 5% by mass or less, or 0.8% by mass or more and 3% by mass or less, and the like.

Examples of the concentration of the polysaccharide in the mixed solution include 0.1 to 40% by mass with respect to the total mass of the mixed solution. The concentration of the polysaccharide in the mixed solution may be, for example, 0.2% by mass or more with respect to the total mass of the mixed solution, 0.5% by mass or more, 0.7% by mass or more, 1% by mass or more, 1.5% by mass or more, 2% by mass or more, 2.5% by mass or more, 3% by mass or more, 3.5% by mass or more, 4% by mass or more, 4.5% by mass or more, or 5% by mass or more. The upper limit value of the concentration of the polysaccharide in the mixed solution is not particularly limited and may be, for example, 35% by mass or less with respect to the total mass of the mixed solution, 30% by mass or less, 25% by mass or less, 20% by mass or less, 15% by mass or less, 10% by mass or less, or 5% by mass or less. It is possible to combine the upper limit value and the lower limit value in any way. Examples of the concentration of the polysaccharide in the mixed solution include 0.5% by mass or more and 30% by mass or less, 1% by mass or more and 20% by mass or less, 1% by mass or more and 15% by mass or less, 2% by mass or more and 10% by mass or less, and the like.

Examples of the concentration of the polyether in the mixed solution include 0.1 to 40% by mass with respect to the total mass of the mixed solution. The concentration of the polyether in the mixed solution may be, for example, 0.2% by mass or more with respect to the total mass of the mixed solution, 0.5% by mass or more, 0.7% by mass or more, 1% by mass or more, 1.5% by mass or more, 2% by mass or more, 2.5% by mass or more, 3% by mass or more, 3.5% by mass or more, 4% by mass or more, 4.5% by mass or more, or 5% by mass or more. The upper limit value of the concentration of the polyether in the mixed solution is not particularly limited and may be, for example, 35% by mass or less with respect to the total mass of the mixed solution, 30% by mass or less, 25% by mass or less, 20% by mass or less, 15% by mass or less, 10% by mass or less, or 5% by mass or less. It is possible to combine the upper limit value and the lower limit value in any way. Examples of the concentration of the polyether in the mixed solution include 0.5% by mass or more and 30% by mass or less, 1% by mass or more and 20% by mass or less, 1% by mass or more and 15% by mass or less, 2% by mass or more and 10% by mass or less, and the like.

The mass ratio of the polysaccharide to the polyether (polysaccharide:polyether) in the mixed solution is not particularly limited and examples thereof include 1:10 to 10:1. The mass ratio of the polysaccharide to the polyether (polysaccharide:polyether) may be 1:9 to 9:1, 2:8 to 8:2, 3:7 to 7:3, 4:6 to 6:4, or 1:1.

The mass ratio of the compound (P) to the polysaccharide (compound (P):polysaccharide) in the mixed solution is not particularly limited and examples thereof include 1:20 to 20:1. The mass ratio of the compound (P) to the polysaccharide (compound (P):polysaccharide) may be 1:20 to 10:1, 1:15 to 8:2, 1:10 to 7:3, 1:7 to 6:4, or 1:5 to 1:1.

The mass ratio of the compound (P) to the polyether (compound (P):polyether) in the mixed solution is not particularly limited and examples thereof include 1:20 to 20:1. The mass ratio of the compound (P) to the polyether (compound (P):polyether) may be 1:20 to 10:1, 1:15 to 8:2, 1:10 to 7:3, 1:7 to 6:4, or 1:5 to 1: l .

The pH of the mixed solution is not particularly limited and may be appropriately adjusted depending on the chemical reaction or the like for which the droplets are used as a reaction site. Examples of the pH of the mixed solution include pH 3 to pH 10.

After obtaining the mixed solution as described above, it is possible to stir the mixed solution to form droplets. It is possible to stir the mixed solution using a stirring device such as a vortex. The droplets formed by this step are liquid-liquid phase-separated droplets. The droplets are dispersed in a mixed solution including the compound (P), a polysaccharide, and a polyether. This step makes it possible to obtain a droplet dispersion liquid in which the droplets are dispersed in a liquid. Therefore, this step may also be said to be a step of producing a droplet dispersion liquid.

The particle size of the droplets formed in this step is approximately 0.1 µm to 500 µm, approximately 0.1 µm to 300 µm, approximately 0.1 µm to 200 µm, approximately 0.1 µm to 100 µm, or approximately 0.1 µm to 80 µm. When the compound (P) is present in the droplet dispersion liquid in the trans form, it is possible for the droplets to maintain the particle size for a long time (for example, 24 hours or more).

### (Other Steps)

The production method of the present embodiment may include other steps. Examples of other steps include a step of irradiating the droplets with light (also referred to below as a "light irradiation step").

### <<Light Irradiation Step>>

The production method of the present embodiment may include a step of irradiating the droplets formed in the droplet formation step with light. The irradiation with light makes it possible to fuse together the droplets at the positions where the light is irradiated. Alternatively, the irradiation with light makes it possible to stop the fusing together of the droplets at the positions where the light is irradiated. The phrase "irradiating the droplets with light" may mean irradiating the droplet dispersion liquid with light. By irradiating a part or the whole of the droplet dispersion liquid with light, it is possible to advance or stop the fusing together of the droplets present in the region where the light is irradiated.

Examples of the wavelength of the irradiation light include (1) a wavelength that causes the compound (P) to undergo cis-conversion, and (2) a wavelength that causes the compound (P) to undergo trans-conversion. The wavelength that causes the compound (P) to undergo cis-conversion or trans-conversion varies depending on the structure of the compound (P) (in particular, the structure of the side chain (b1)). Therefore, it is possible to select the wavelength of light used in the light irradiation step according to the structure of the compound (P).

By irradiating droplets with irradiation light having a wavelength that causes compound (P) to undergo cis-conversion, it is possible to advance the fusing together of the droplets present in the region irradiated with light. By irradiating droplets with irradiation light having a wavelength that causes compound (P) to undergo cis-conversion, the compound (P) present around the droplets undergoes cis-conversion. This is considered to cause the liquid around the droplets to undergo solation and cause fusion of the droplets to advance.

For example, when compound (P) has a side chain represented by Formula (b1-1-1) as the side chain (b1), examples of the wavelength of irradiation light that causes compound (P) to undergo cis-conversion include 400 nm or less. Examples of the wavelength of the irradiation light include 200 nm to 400 nm, 250 nm to 390 nm, 300 nm to 380 nm, 320 nm to 370 nm, or 330 nm to 360 nm and, for example, it is possible to use 350 nm irradiation light. For example, when the compound (P) has a side chain represented by Formula (b1-1-7) as the side chain (b1), examples of the wavelength of the irradiation light that causes compound (P) to undergo cis-conversion include 400 nm to 700 nm. Examples of the wavelength of the irradiation light include 450 nm to 650 nm, 500 nm to 600 nm, 510 nm to 580 nm, 520 nm to 570 nm, or 530 nm to 560 nm and, for example, it is possible to use 540 nm irradiation light.

By irradiating droplets with irradiation light having a wavelength that causes the compound (P) to undergo trans-conversion, it is possible to stop the fusing together of the droplets present in the region irradiated with light. By irradiating the droplets with irradiation light having a wavelength that causes the compound (P) to undergo trans-conversion, the compound (P) present around the droplets undergoes trans-conversion. This is considered to cause the liquid around the droplets to undergo gelation and stop the fusion of the droplets.

For example, when the compound (P) has a side chain represented by Formula (b1-1-1) or (b1-1-7) as the side chain (b1), examples of the wavelength of the irradiation light that causes the compound (P) to undergo trans-conversion include 410 nm to 600 nm. Examples of the wavelength of the irradiation light include 420 nm to 550 nm, 420 nm to 500 nm, 430 nm to 480 nm, 430 nm to 470 nm, or 430 nm to 460 nm and, for example, it is possible to use 450 nm irradiation light.

The time for which the droplets are irradiated with light is not particularly limited. When the irradiation time is increased, the photoisomerization of the compound (P) advances. For example, when a droplet dispersion liquid including the trans form of the compound (P) is irradiated with light having a wavelength that causes the compound (P) to undergo cis-conversion, the fusing together of the droplets progresses when the irradiation time increases, which makes it possible to form larger droplets. For example, when a droplet dispersion liquid including the cis form of the compound (P) is irradiated with light having a wavelength that causes the compound (P) to undergo trans-conversion, increasing the irradiation time makes it possible to further suppress the rate at which the fusing together of the droplets advances. It is possible to control the fusion state of the droplets by adjusting the light irradiation time. The light irradiation time may be long enough to cause photoisomerization of the compound (P). Examples of the light irradiation time include approximately 0.1 seconds to 15 minutes. Examples of the light irradiation time include 0.5 seconds or more, 1 second or more, 5 seconds or more, 10 seconds or more, and the like. Examples of the light irradiation time include 0.1 seconds or more and 15 minutes or less, 0.5 seconds or more and 15 minutes or less, 1 second or more and 15 minutes or less, 5 seconds or more and 15 minutes or less, 10 seconds or more and 15 minutes or less, and the like. To maintain the photoisomerized state (cis or trans) caused by the light irradiation, the droplet dispersion liquid may be continuously irradiated with light of the same wavelength, or the droplet dispersion liquid may be maintained in a dark place after the light irradiation.

Examples of the radiant flux of the irradiation light include 10 W or more, preferably 20 W or more, and more preferably 30 W or more. When the droplets are irradiated with light, it is considered that a part of the irradiation light is used for the photoisomerization of the compound (P) around the droplets and a part is transmitted through the droplets. Therefore, the photoisomerization rate of the compound (P) is not necessarily proportional to the amount of energy of the irradiation light. It is possible for the radiant flux of the irradiation light to be set to, for example, 200 W or less or 100 W or less, taking into account the power consumption.

The light irradiation step is preferably performed by placing the droplet dispersion liquid in a container having a depth of approximately 0.1 mm to 10 mm. The depth of the container is preferably approximately 0.5 mm to 5 mm, more preferably approximately 0.5 mm to 3 mm, even more preferably 0.5 mm to 2 mm, and particularly preferably 1 mm. When the depth of the container is in the above-described preferable range, overlap between the droplets in the vertical direction is suppressed, making it easier for the irradiation light to hit all droplets present at the desired position. Examples of containers include transparent cells having the depths described above, transparent channel devices having the channel depths described above, and the like.

For example, it is possible to advance the fusing together of the droplets by producing droplets using the compound (P) in the trans form and irradiating the droplets with light having a wavelength that that causes the compound (P) to undergo cis-conversion. Thereafter, it is possible to stop the fusing together of the droplets by irradiating the droplets with light having a wavelength that trans-converts the compound (P). Alternatively, after producing the droplets, it is possible to stably store the droplets for a long period of time in the state at the time of production by irradiating the droplets with light having a wavelength that causes the compound (P) to undergo trans-conversion.

According to the production method of the present embodiment, it is possible to obtain droplets that are stable for a long time. It is possible to fuse together the droplets in a position-selective manner at any timing by light irradiation. It is possible to use the droplets obtained by the production method of the present embodiment as a reaction site for chemical reactions and to apply the droplets to single-molecule sensing technologies.

### <Reaction Method>

The fifth aspect of the present disclosure is a reaction method. The reaction method of the present embodiment includes (a) a step of stirring a solution including the compound (P), a polysaccharide, a polyether, and a first reactant to produce a first droplet including the first reactant, (b) a step of stirring a solution including the compound (P), a polysaccharide, a polyether, and a second reactant to produce a second droplet including the second reactant, (c) a step of mixing the first droplet and the second droplet, and (d) a step of irradiating the mixed first droplet and second droplet with light to fuse the first droplet and the second droplet to produce a reaction site droplet including the first reactant and the second reactant.

### (Step (a))

In step (a), a solution including the compound (P), a polysaccharide, a polyether, and the first reactant (also referred to below as "first mixed solution") is stirred to produce a first droplet including the first reactant.

The first mixed solution is a solution in which the first reactant is added to the mixed solution described above in <Method for Producing Droplets>. It is preferable to use the compound (P) in which the side chain (b1) is in the trans form. The first reactant is not particularly limited and it is possible to select any reactant depending on the purpose. Examples of the first reactant include organic synthetic compounds such as low molecular weight compounds, fluorescent dyes, radical generators, and synthetic polymer compounds; biomolecules such as amino acids, nucleotides, sugars, vitamins, peptides, proteins, and nucleic acids; inorganic compounds such as inorganic nanoparticles, inorganic anions, inorganic cations, inorganic salts, and aggregates thereof; cells such as animal cells, plant cells, fungi, algae cells, and protozoa cells; extremely small structures such as phages, viruses, and molecular aggregates; and cell aggregates such as organoids and spheroids, without being limited thereto.

It is possible to obtain a first mixed solution by adding and dissolving the first reactant in the above-described mixed solution. Next, by stirring the first mixed solution, it is possible to produce a first droplet in which the first reactant is encapsulated in the droplet.

In step (a), a polysaccharide labeled using a fluorescent dye or the like may be used as the polysaccharide. By using the fluorescently labeled polysaccharide, when a mixed droplet solution is produced in step (c) described below, it is possible to identify the first droplet by using the fluorescent dye as an indicator.

By this step, it is possible to obtain a first droplet dispersion liquid in which the first droplets are dispersed in a liquid.

### (Step (b))

In step (b), a solution including the compound (P), a polysaccharide, a polyether, and the second reactant (also referred to below as the "second mixed solution") is stirred to produce a second droplet including the second reactant.

The second mixed solution is a solution in which the second reactant is added to the mixed solution described above in <Method for Producing Droplets>. The second reactant is not particularly limited and it is possible to select any reactant according to the purpose. Examples of the second reactant may be the same as the first reactant.

It is possible to obtain the second mixed solution by adding and dissolving the second reactant in the above-described mixed solution. Next, it is possible to produce a second droplet in which the second reactant is encapsulated in the droplet by stirring the second mixed solution.

In step (b), a polysaccharide labeled using a fluorescent dye or the like may be used as the polysaccharide. By using a fluorescently labeled polysaccharide, It is possible to identify the second droplet by using the fluorescent dye as an indicator when the mixed droplet solution is produced in step (c) described below. When a polysaccharide labeled using a fluorescent dye is used in step (a), in step (b), it is preferable to use a different type of fluorescent dye from the fluorescent dye used in step (a).

By this step, it is possible to obtain a first droplet dispersion liquid in which the second droplets are dispersed in the liquid.

### (Step (c))

In step (c), the first droplets and the second droplets are mixed.

It is possible to produce the mixed droplet solution by mixing the solution including the first droplets produced in step (a) and the solution including the second droplets produced in step (b). The mixing ratio of the first droplets and the second droplets is not particularly limited. For example, the solution including the first droplets and the solution including the second droplets may be mixed in equal amounts. The mixed droplet solution is a mixed droplet dispersion liquid in which the first droplets and the second droplets are dispersed in a liquid. The solution including the first droplets is the first droplet dispersion liquid produced in step (a). The solution including the second droplets is the second droplet dispersion liquid in step (b).

### (Step (d))

In step (d), the droplet dispersion liquid including the first droplets and second droplets mixed in step (c) is irradiated with light to fuse the first droplets and second droplets, thereby producing a reaction site droplet including the first reactant and second reactant.

The wavelength of the irradiation light is a wavelength of light that causes the compound (P) to undergo cis-conversion and it is possible to select this wavelength according to the structure of the compound (P). Examples of such irradiation light include the same examples as for the (light irradiation step) described above in <Method for Producing Droplets>.

It is preferable that the light irradiation is performed at a position where the first droplets and second droplets are present in close proximity. The first droplets and second droplets in close proximity are fused by the light irradiation, which makes it possible to obtain a reaction site droplet including the first reactant and second reactant. The term "reaction site droplet" is a droplet including the first reactant and the second reactant. Since the reaction site droplet encapsulates the first reactant and the second reactant, it is possible to use the reaction site droplet as a reaction site for reacting the first reactant and the second reactant.

FIG. 2 is a schematic diagram showing an embodiment of step (c) and step (d). In the example of FIG. 2, the first droplet and the second droplet are mixed using a channel device including a first channel 10, a second channel 20, and a third channel 30. In the channel device of FIG. 2, the first channel 10, the second channel 20, and the third channel 30 form a three-way junction at the junction point A. A first droplet 1 moves through the first channel 10 and is sent out from the junction point A to the third channel 30. A second droplet 2 moves through the second channel 20 and is sent out from the junction point A to the third channel 30. The first droplet 1 and the second droplet 2 join at the junction point A and the first droplet 1 and the second droplet 2 are mixed in the third channel 30. By synchronizing the timing at which the first droplet 1 is sent out from the first channel 10 to the third channel 30 with the timing at which the second droplet 2 is sent out from the second channel 20 to the third channel 30, it is possible to make the first droplet 1 and the second droplet 2 be present in close proximity in the third channel 30. The first droplet 1 and the second droplet 2 move through the third channel 30 in a state of close proximity to each other. When these droplets reach point B and the droplets are irradiated with light having a wavelength that causes the compound (P) to undergo cis-conversion, these droplets fuse to form a reaction site droplet 3. The reaction site droplet 3 inherits the reactants enclosed in the first droplet and second droplet and thus encloses the first reactant and second reactant. Therefore, it is possible to use the reaction site droplet 3 as a reaction site for reacting the first reactant and second reactant. Examples of the irradiation time of the light having a wavelength that causes the compound (P) to undergo cis-conversion include approximately 0.1 seconds to 15 minutes, preferably approximately 0.1 seconds to 1 minute.

After the reaction site droplet 3 is produced by step (d), the reaction site droplet 3 may be irradiated with light having a wavelength that causes the compound (P) to undergo trans-conversion. It is possible to select the wavelength that causes the compound (P) to undergo trans-conversion according to the structure of the compound (P). For example, in FIG. 2, when the reaction site droplet 3 reaches point C, the reaction site droplet 3 is irradiated with light having a wavelength that causes the compound (P) to undergo trans-conversion. This makes it possible to stably maintain the reaction site droplet 3. Examples of the irradiation time of the light having a wavelength that causes the compound (P) to undergo trans-conversion include approximately 0.1 seconds to 15 minutes, preferably approximately 0.1 seconds to 1 minute.

### (Other Steps)

The method of the present embodiment may include other steps in addition to the above-described steps (a) to (d). The other steps may include, for example, (e) a step of incubating the reaction site droplet and (f) a step of analyzing a reaction product.

### <<Step (e)>>

The method of the present embodiment may include a step of incubating the reaction site droplet. By incubating the reaction site droplet, it is possible to react the first reactant and the second reactant in the reaction site droplet. It is possible to appropriately set the incubation temperature of the reaction site droplet depending on the type of reaction without being particularly limited. The incubation time is also not particularly limited and it is possible to appropriately set the incubation time depending on the type of reaction.

### <<Step (f)>>

The method of the present embodiment may include a step of analyzing a reaction product. The analysis of the reaction product may be performed without destroying the reaction site droplet or may be performed by destroying the reaction site droplet and extracting the reaction product. Alternatively, a droplet further including an analysis reagent may be fused to the reaction site droplet to produce an analysis droplet including the reaction product and the analysis reagent. It is possible to analyze the reaction product by reacting the reaction product with the analysis reagent in the analysis droplet. When the analysis operation is multi-stage, the analysis operation may be progressed by producing droplets including analysis reagents for each stage and fusing these droplets with the reaction site droplet in order.

To perform a reaction in a droplet, a technology is required to fuse a droplet including a substrate with a droplet including a reactant at a desired timing and at a desired position. According to the method of the present embodiment, it is possible to fuse droplets at any timing by irradiation with light having a wavelength that causes the compound (P) to undergo cis-conversion and to use the droplets as a reaction site. Since it is possible to produce extremely small, micro-scale droplets by liquid-liquid phase separation, it is possible to provide an extremely small reaction site. It is possible to apply the method of the present embodiment to single molecule sensing.

### <Droplet Formation Composition>

The sixth aspect of the present disclosure is a droplet formation composition.

The droplet formation composition of the present embodiment includes the compound (P), a polysaccharide, and a polyether. The droplet formation composition is the same as the "mixed solution" described in <Method for Producing Droplets>.

### <Droplet Control Agent>

The seventh aspect of the present disclosure is a droplet control agent. The droplet control agent of the present embodiment includes the compound (P). The droplet control agent may consist of the compound (P). In the present specification, the phrase "controlling droplets" encompasses stably maintaining the size (particle size) of droplets, controlling the advancing of and stopping the fusing together of the droplets, and controlling the size (particle size) of the droplets by controlling the fusing together of the droplets.

The compound (P) is the same as described above. The droplet control agent may include an optional component in addition to the compound (P). Examples of the optional component include water or a buffer solution. Examples of the water or buffer solution include the same examples as for the above-described <Method for Producing Droplets>.

It is possible to add the droplet control agent to a mixed solution for producing droplets including a polysaccharide and a polyether when producing liquid-liquid phase-separated droplets. When used during droplet production, the compound (P) included in the droplet control agent is preferably in the trans form. Alternatively, the droplet control agent may be added to a droplet dispersion liquid after producing liquid-liquid phase-separated droplets using a mixed solution for producing droplets including a polysaccharide and a polyether. In this case, the compound (P) included in the droplet control agent is preferably in the trans form. By having a droplet control agent including the compound (P) in the trans form present in the droplet dispersion liquid, it is possible to stably maintain the droplets.

By irradiating the droplet dispersion liquid including the droplet control agent with light having a wavelength that causes the compound (P) to undergo cis-conversion, it is possible to advance the fusing together of the droplets present in the region irradiated with the light. Furthermore, by irradiating the droplet dispersion liquid with light having a wavelength that causes the compound (P) to undergo trans-conversion, it is possible to stop the fusing together of the droplets present in the region irradiated with the light.

In this manner, according to the droplet control agent of the present embodiment, it is possible to control the fusion of the droplets and control the size of the droplets by irradiation with light. In addition, according to the droplet control agent of the present embodiment, it is possible to stably maintain droplets of a desired size.

### <Method for Controlling Droplets>

The seventh aspect of the present disclosure is a method for controlling droplets. The droplet control method of the present embodiment includes causing droplets formed by stirring a solution including a polysaccharide and a polyether to coexist with the compound (P).

The droplets controlled by the method of the present embodiment are liquid-liquid phase-separated droplets. It is possible to produce droplets by the droplet production method of the third aspect described above. The compound (P) may be added to the mixed solution when producing the droplets or may be added to the droplet dispersion liquid after the droplets are produced.

The method of the present embodiment may further include irradiating the droplets coexisting with the compound (P) with light. Examples of the wavelength of the light to be irradiated include (1) a wavelength that causes the compound (P) to undergo cis-conversion or (2) a wavelength that causes the compound (P) to undergo trans-conversion. By irradiating the droplet dispersion liquid with light of the above-described wavelength (1) or (2) in a state where the droplets and the compound (P) coexist, it is possible to control the fusing together of the droplets. For example, by irradiating the droplet dispersion liquid with light having a wavelength that causes the compound (P) to undergo cis-conversion, it is possible to advance the fusing together of the droplets. This makes it possible to increase the size of the droplets. Alternatively, it is possible to fuse together droplets encapsulating different substances to produce droplets encapsulating a plurality of types of substances. When the desired droplets are obtained, it is possible to stop the fusion of the droplets by irradiating the droplet dispersion liquid with light having a wavelength that causes the compound (P) to undergo trans-conversion. This makes it possible to stably maintain the desired droplets.

It is possible to appropriately select the wavelengths (1) and (2) above depending on the structure of the compound (P). It is possible to appropriately set the light irradiation time depending on the target droplet size. Examples of the light irradiation time include approximately 0.1 seconds to 15 minutes.

### Examples

An explanation will be given of the present invention based on Examples. However, the embodiments of the present invention are not limited to the description of these Examples.

### [Example 1]

### <Synthesis Example of Compound (P1)>

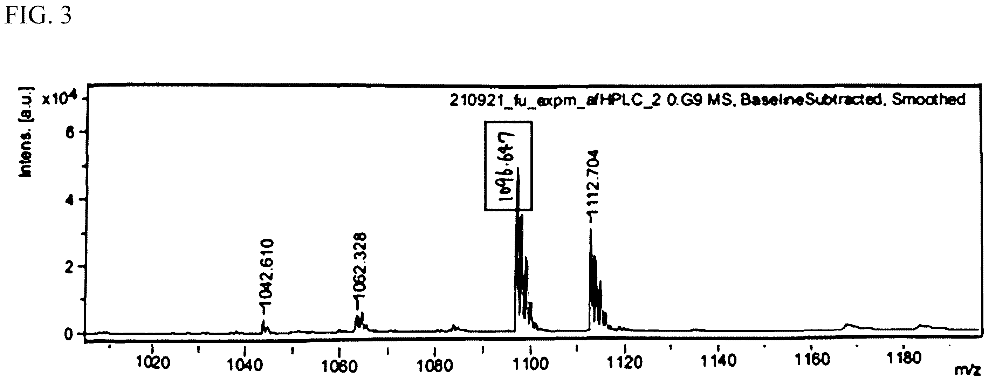

The above-described compound (P1) was synthesized by solid-phase peptide synthesis. The phenylazophenylalanine used in the peptide synthesis was purchased from Tokyo Chemical Industry Co., Ltd., and the other amino acids (arginine, aspartic acid, alanine, and methionine) were purchased from Watanabe Chemical Industry Co., Ltd. The synthesized compound (P1) was analyzed using matrix-assisted laser desorption/ionization (MALDI) and the results are shown in FIG. 3. A peak (observed value 1096.65) derived from the molecular weight (theoretical value 1096.55) of a proton adduct of the compound (P1) was observed. This result confirmed that the compound (P1) was obtained.

### <Photoresponsiveness of Compound (P1)>

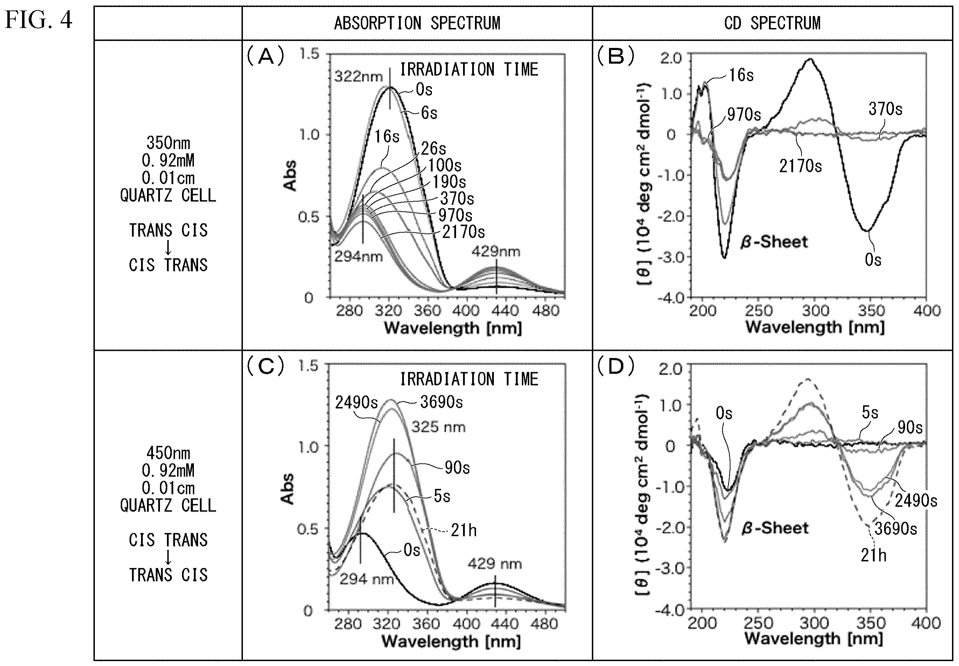

The compound (P1) was dispersed in a D-MEM buffer solution including 4 mM HEPES to produce a dispersion liquid including 1 wt*%* of the compound (P1). This dispersion liquid was placed in a transparent cell having a depth of 1 mm and irradiated with light using a fluorescence microscope (Olympus IX-73). A U-HGLGPS (brightness: 130 W) was used as the light source for the fluorescence microscope and light was irradiated with a brightness output setting of 25*%*. The absorption spectrum and circular dichroism (CD) spectrum of the dispersion liquid after light irradiation were measured by ultraviolet-visible light spectroscopy. The measurements were performed at room temperature (25°C). The results are shown in FIG. 4. (A shows the absorption spectrum when irradiated with 350 nm light. (B) shows the CD spectrum when irradiated with 350 nm light. (C) shows the absorption spectrum when irradiated with 450 nm light. (D) shows the CD spectrum when irradiated with 450 nm light.

When irradiated with 350 nm light, a decrease in the absorption peak near 320 nm and an increase in the absorption peak near 430 nm were observed (FIG. 4 (A)). These results confirmed that the azobenzene structure in the compound (P1) was photoisomerized from the trans form to the cis form. When irradiated with 350 nm light for 2170 seconds, a decrease in the peak near 220 nm was confirmed in the CD spectrum (FIG. 4 (B)). These results suggested that the compound (P1) formed a β-sheet.

When irradiated with 450 nm light, an increase in the absorption peak near 320 nm and a decrease in the peak near 430 nm were observed (FIG. 4 (A)). These results confirmed that the azobenzene structure in compound (P1) was photoisomerized from the cis form to the trans form.

The above-described results confirmed that the compound (P1) was reversibly isomerized from the trans form to the cis form and from the cis form to the trans form when irradiated with light.

The results in FIG. 4(C) confirmed that the peak near 220 nm was decreased when 350 nm light was irradiated. The results in FIG. 4(D) confirmed that the peak near 220 nm was increased when 450 nm light was irradiated. These results suggested that the trans form of the compound (P1) formed a β-sheet.

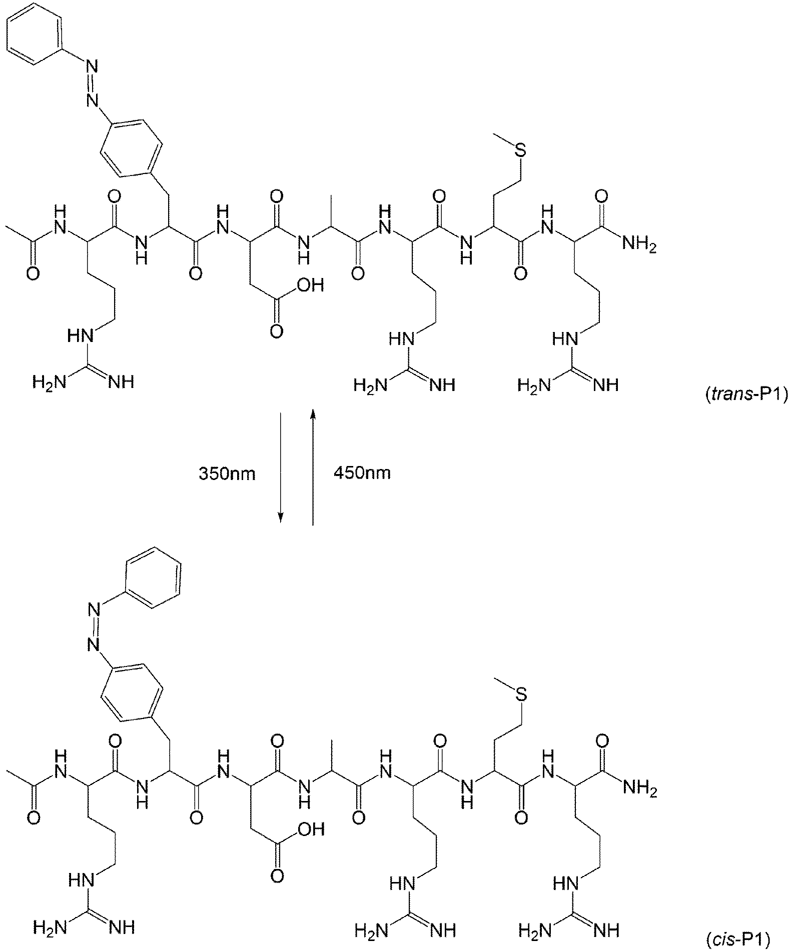

### <Droplet Production Example 1>

A mixed aqueous solution including 5 wt*%* dextran (550 kDa; DEX550k), 0.002 wt*%* rhodamine-DEX550k, and 5 wt*%* polyethylene glycol (35 kDa; PEG35k) was produced in a microtube. Next, the compound (P1) was added to the mixed aqueous solution to be 1 wt*%* thereof, dissolved, and stirred for 1 minute using a vortex. Due to this, liquid-liquid phase-separated droplets were produced.

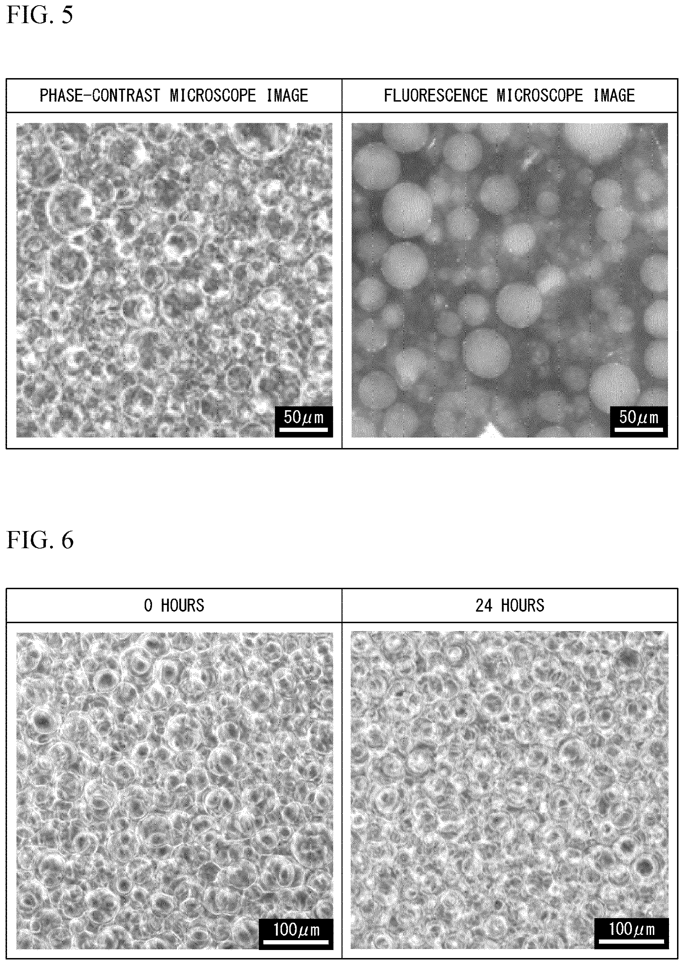

FIG. 5 shows a phase-contrast microscope image and a fluorescence microscope image of the produced droplets. These images confirmed the formation of droplets. Furthermore, since rhodamine fluorescence was observed in the droplets, it was confirmed that the droplets were mainly composed of DEX550k.

### <Evaluation of Droplet Stability (1)>

Liquid-liquid phase-separated droplets were produced by the same method as in <Droplet Production Example 1>. After production of the droplets, the droplets were left to stand for 24 hours and the state of the droplets was observed using a phase-contrast microscope.

FIG. 6 shows phase-contrast microscope images of the droplets immediately after production (0 hours) and 24 hours after production. After 24 hours, the droplets were observed to be the same as immediately after production. This result confirmed that the fusing together of the droplets hardly occurred and that the droplets were stably maintained for 24 hours or more.

### <Evaluation of Droplet Stability (2)>

Liquid-liquid phase-separated droplets were produced by the same method as in <Droplet Production Example 1>. After production of the droplets, the droplets were left to stand for 16 days and the state of the droplets was observed using a phase-contrast microscope.

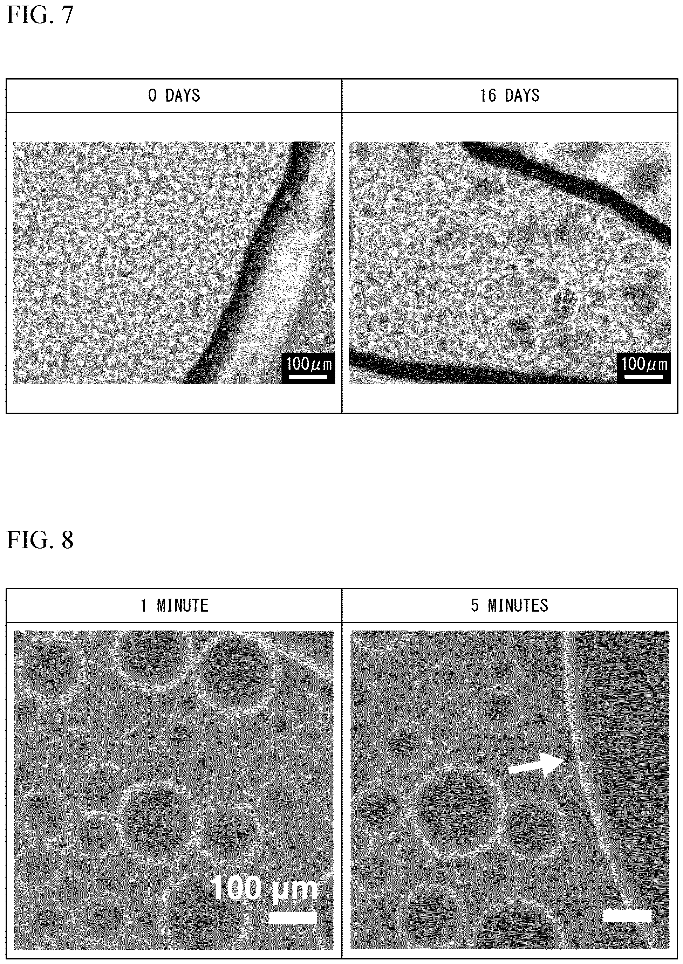

FIG. 7 shows phase-contrast microscope images of the droplets immediately after production (day 0) and 16 days after production. It was confirmed that the droplets were stably maintained even after 16 days.

### <Evaluation of Droplet Stability (Comparative Example)>

A mixed aqueous solution including 5 wt*%* DEX550k and 5 w*%* PEG35k was produced in a microtube. The mixed aqueous solution was stirred using a vortex to produce liquid-liquid phase-separated droplets. After production of the droplets, the droplets were left to stand for 5 minutes and the state of the droplets was observed using a phase-contrast microscope.

FIG. 8 shows microscope images of the droplets taken 1 minute and 5 minutes after production. Five minutes after the droplets were produced, it was confirmed that the droplets had fused together to form a large droplet (indicated by the white arrow).

### <Droplet Fusion by Position-Selective Light Irradiation>

A mixed aqueous solution including 5 wt*%* DEX550k and 5 w*%* PEG35k was produced in a microtube. Next, the compound (P1) was added to the mixed aqueous solution to be 1 wt*%* thereof, dissolved, and stirred for 1 minute using a vortex. Due to this, liquid-liquid phase-separated droplets were produced. The produced droplets were position-selectively irradiated with 350 nm light for 6 minutes. The droplet dispersion liquid was placed in a transparent cell having a depth of 1 mm and light irradiation was performed using a fluorescence microscope (Olympus IX-73). A U-HGLGPS (brightness: 130 W) was used as the light source for the fluorescence microscope and light was irradiated with a brightness output setting of 25*%*.

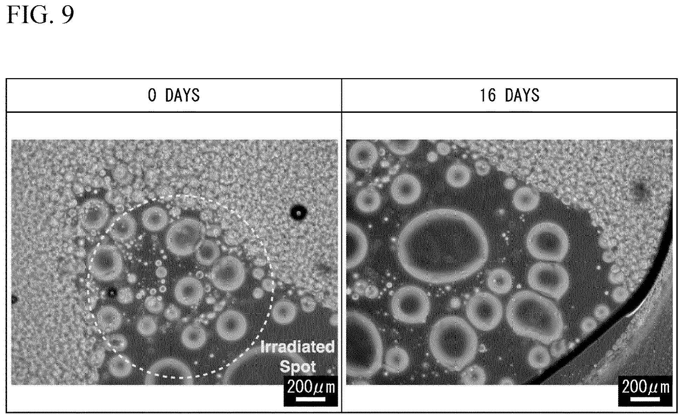

FIG. 9 shows microscope images of droplets immediately after the start of light irradiation and 6 minutes after the start of light irradiation. In FIG. 9, the portion surrounded by a dotted circle is the light irradiated spot. It was confirmed that the fusing together of the droplets advanced in the light irradiated spot and a larger droplet was formed after 6 minutes. This result confirmed that it is possible to fuse the droplets at any position at any timing by light irradiation.

### <Observation of Localization of Compound (P1)>

To observe the localization of the compound (P), the compound (P1) was labeled using Cy5. A mixed aqueous solution including 5 wt*%* dextran (550 kDa; DEX550k) and 5 wt*%* polyethylene glycol (35 kDa; PEG35k) was produced in a microtube. Next, the Cy5-labeled compound (P1) was added to the mixed aqueous solution to be 1 wt*%* thereof, dissolved, and stirred for 1 minute using a vortex. Due to this, liquid-liquid phase-separated droplets were produced. The droplets were irradiated with 350 nm light for 5 seconds. The droplet dispersion liquid was placed in a transparent cell having a depth of 1 mm and light irradiation was performed using a fluorescence microscope (Olympus IX-73). A U-HGLGPS (brightness: 130 W) was used as the light source for the fluorescence microscope and light was irradiated with a brightness output setting of 25*%*. Before and after light irradiation, the droplets were observed using a phase-contrast microscope (Olympus IX-73 connected to a phase-contrast light source) and a fluorescence microscope (Olympus IX-73 connected to a fluorescence light source

### U-HGLGPS).

The upper part of FIG. 10 shows a phase-contrast microscope image and a fluorescence microscope image of the droplets before light irradiation. The lower part of FIG. 10 shows a phase-contrast microscope image and a fluorescence microscope image of the droplets after irradiation with 350 nm light. In the fluorescence microscope image, the red fluorescence was localized outside the droplets. This confirmed that the compound (P1) was localized outside the droplets. After the light irradiation, droplets which were larger than before the light irradiation were observed. This was considered to be due to the advancing of the fusing together of the droplets.

Before irradiation with 350 nm light, the droplet dispersion liquid was in a gel state, but after irradiation with 350 nm light, the droplet dispersion liquid changed to a sol state. When irradiated with 350 nm light, the compound (P1) changes from the trans form to the cis form (refer to FIG. 4). From the above, it was considered that the compound (P1) in the trans form is present in a fibrous state outside the droplets, which causes the droplet dispersion liquid to enter a gel state. It was considered that the droplet dispersion liquid being in a gel state stably maintains the individual droplets. On the other hand, it was considered that, in the compound (P1) in the cis form, the fibrous structure is broken and is dispersed outside the droplets, which causes the droplet dispersion liquid to enter a sol-state. It was considered that the droplet dispersion liquid entering a sol-state decreases the external force maintaining the individual droplets, which progresses the fusing together of the droplets.

### <Control of Droplets by Light Irradiation>

A mixed aqueous solution including 5 wt*%* dextran (550 kDa; DEX550k) and 5 wt*%* polyethylene glycol (35 kDa; PEG35k) was produced in a microtube. Next, the labeled compound (P1) was added to the mixed aqueous solution to be 1 wt*%* thereof, dissolved, and stirred for 1 minute using a vortex. Due to this, liquid-liquid phase-separated droplets were produced. The droplets were subjected to any of the following light irradiation operations (1) to (3). The light irradiation was performed in the same manner as described above.
(1) No light irradiation.
(2) Irradiation with 350 nm light for 5 seconds.
(3) Irradiation with 350 nm light for 5 seconds, followed by irradiation with 550 nm light for 5 seconds.

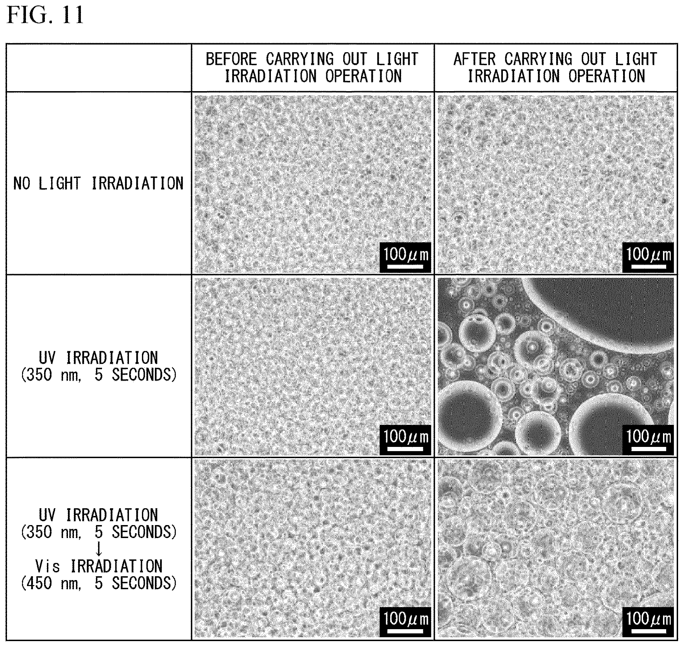

The droplets were observed under a phase-contrast microscope before and after carrying out the light irradiation operation under the above-described conditions. The results are shown in FIG. 11. In (1), an image "before carrying out the light irradiation operation" was taken and then an image "after carrying out the light irradiation operation" was taken within 1 minute. No light irradiation was performed between "before carrying out the light irradiation operation" and "after carrying out the light irradiation operation". In (2), an image "after carrying out the light irradiation operation" was taken immediately after 5 seconds of irradiation with 350 nm light. In (3), 350 nm light was irradiated for 5 seconds, immediately followed by irradiating 550 nm light for 5 seconds, and an image "after carrying out the light irradiation operation" was taken immediately after the 550 nm light was irradiated for 5 seconds.

(1) In the absence of light irradiation, no change was seen in the size of the droplets. (2) In the case of irradiating 350 nm light for 5 seconds, the number of droplets decreased and the size of the droplets significantly increased after carrying out the light irradiation operation compared to before carrying out the light irradiation operation. (3) When irradiated with 350 nm light for 5 seconds and then irradiated with 550 nm light for 5 seconds, the number of droplets decreased and the size of the droplets increased after carrying out the light irradiation operation compared to before carrying out the light irradiation operation. However, compared to the case of (2), the size of the droplets was smaller.

FIG. 12 is a schematic diagram showing state changes of the droplet dispersion liquid that are estimated to occur due to the light irradiation operations (1) to (3) described above. (1) In the absence of light irradiation, the compound (P1) remains in the trans form and the droplet dispersion liquid is in a gel state. Therefore, the droplets are stably maintained. In the case of (2), when irradiated with 350 nm light, the compound (P1) changes from the trans form to the cis form. As a result, the droplet dispersion liquid undergoes solation and the fusing together of the droplets progresses. In the case of (3), when irradiated with 350 nm light, the compound (P1) changes from the trans form to the cis form, and when subsequently irradiated with 550 nm light, the compound (P1) returns from the cis form to the trans form. While compound (P1) is in the cis form, the droplet dispersion liquid undergoes solation and the fusing together of the droplets progresses. However, when the compound (P1) returns to the trans form, the droplet dispersion liquid undergoes gelation and the fusing together of the droplets stops or is suppressed.

These results confirmed that it is possible to control the droplet size by adjusting the wavelength of the light irradiation and the light irradiation time.

### <TEM Analysis of Compound (P1)>

The trans form of the compound (P3) was dispersed in a D-MEM buffer solution (4 mM HEPES, containing 1 wt*%* NaHCO₃, pH 8.5) to produce a dispersion liquid including 1.0 wt*%* of the compound (P1). This dispersion liquid was incubated for 3 days in a dark place at 37°C in a 5*%* CO₂ atmosphere. Thereafter, the dispersion liquid was irradiated with 450 nm light and analyzed with a transmission electron microscope (JEOL JEM-1400).

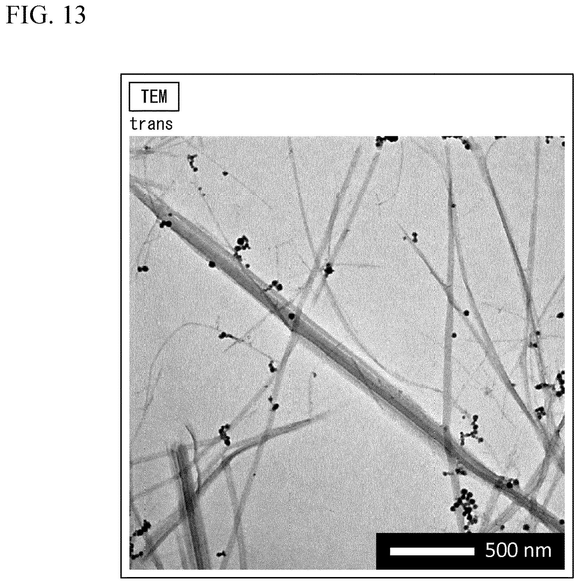

The results of the TEM analysis are shown in FIG. 13. TEM images confirmed that the trans form of the compound (P1) formed a structure in a fibrous state.

### <Rheological Evaluation>

The trans form of the compound (P1) was dispersed in a D-MEM buffer solution (4 mM HEPES, containing 1 wt*%* NaHCO₃, pH 8.5) to produce a dispersion liquid including 1.0 wt*%* of the compound (P1). This dispersion liquid was incubated for 3 days in a dark place at 37°C in a 5*%* CO₂ atmosphere. Thereafter, the storage modulus G' and loss modulus G" were measured using a rotational rheometer (Malvern Panalytical, Kinexus lab+).

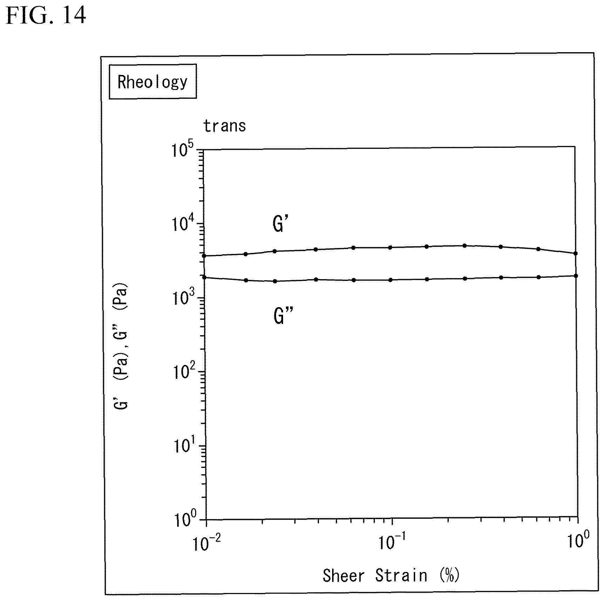

The results are shown in FIG. 14. In the trans form of the compound (P1), the value of the storage modulus G' exhibited a value greater than the loss modulus G", thus confirming that a gel was formed.

### <IR Analysis of Compound (P1)>

The compound (P1) was dispersed in a D-MEM buffer solution (4 mM HEPES, containing 1 wt*%* NaHCO₃, pH 8.5) to produce a dispersion liquid including 1.0 wt*%* of the compound (P1). This dispersion liquid was incubated for 3 days in a dark place at 37°C in a 5*%* CO2 atmosphere. Thereafter, the dispersion liquid was irradiated with 450 nm light and analyzed by absorption spectrum analysis using infrared absorption spectroscopy analysis (IR) (JASCO FTIR-6100).

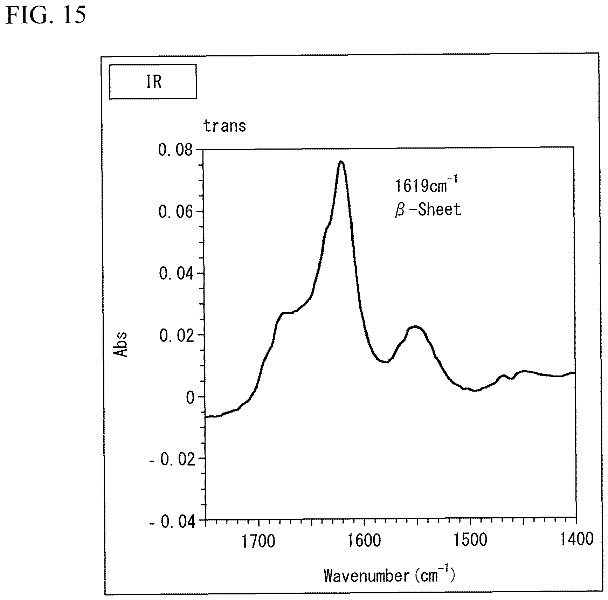

The results of the IR analysis are shown in FIG. 15. A peak at 1619 cm⁻¹ was confirmed for the trans form of the compound (P1). This result suggests that the trans form of the compound (P1) forms a β-sheet.

### <Reversible Gel-Sol Transition>

The compound (P1) was dispersed in a D-MEM buffer solution (4 mM HEPES, containing 1 wt*%* NaHCO₃, pH 8.5) to produce a dispersion liquid including 1.0 wt*%* of the compound (P1). The dispersion liquid was placed in a test tube to observe the gel-sol transition caused by light irradiation. Incubation after the start of light irradiation was performed at 37°C in a 5*%* CO₂ atmosphere.

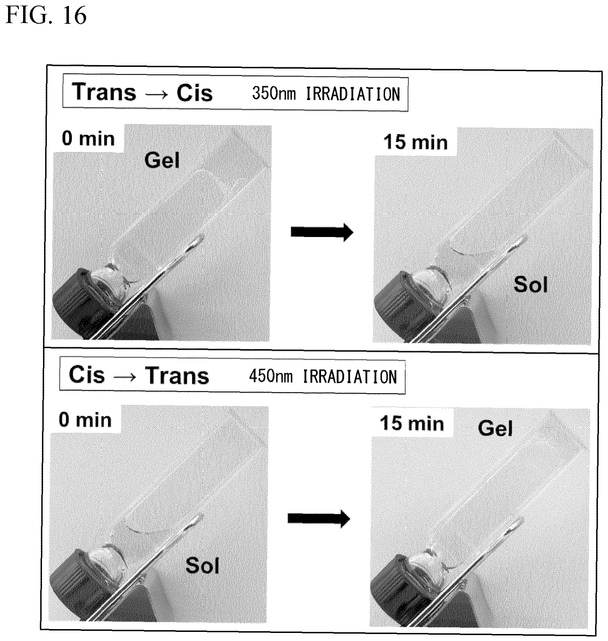

The results are shown in FIG. 16. The dispersion liquid including the compound (P1) in the trans form underwent gelation at the start of light irradiation (0 min). When the dispersion liquid was irradiated with 350 nm light for 15 minutes, the dispersion liquid underwent solation. The dispersion liquid including the compound (P1) in the cis form underwent solation at the start of light irradiation (0 min). When the dispersion liquid was irradiated with 450 nm light for 15 minutes, the dispersion liquid underwent gelation.

### [Example 2]

### <Synthesis Example of Compound (P2)>

The above-described compound (P2) was synthesized by solid-phase peptide synthesis. The phenylazophenylalanine used in the peptide synthesis was purchased from Tokyo Chemical Industry Co., Ltd., and the other amino acids (arginine, aspartic acid, alanine, methionine) were purchased from Watanabe Chemical Industry Co., Ltd. The synthesized compound (P1) was analyzed using matrix-assisted laser desorption/ionization (MALDI). A peak (observed value 1096.59) derived from the molecular weight (theoretical value 1096.55) of the proton adduct of the compound (P2) was observed. This result confirmed that the compound (P2) was obtained.

### <Fusion of Droplets by Light Irradiation>

A mixed aqueous solution including 5 wt*%* DEX550k and 5 w*%* PEG35k was produced in a microtube. Next, the compound (P2) was added to the mixed aqueous solution to be 1 wt*%* thereof, dissolved, and stirred for 1 minute using a vortex. Due to this, liquid-liquid phase-separated droplets were produced. The produced droplets were irradiated with 350 nm light for 5 seconds. The droplet dispersion liquid was placed in a transparent cell having a depth of 1 mm and light irradiation was performed using a fluorescence microscope (Olympus IX-73). A U-HGLGPS (brightness: 130 W) was used as the light source for the fluorescence microscope and light was irradiated with a brightness output setting of 25*%*.

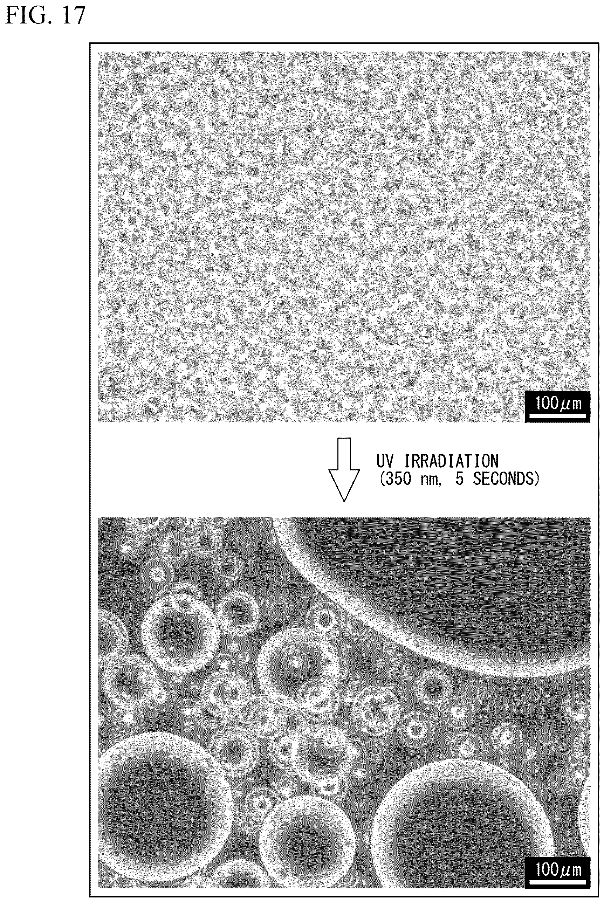

FIG. 17 shows microscope images of the droplets immediately after the start of light irradiation and 5 seconds after the start of light irradiation. It was confirmed that the fusing together of the droplets advanced due to the light irradiation and larger droplets were formed. This was considered to be due to the photoisomerization of the compound (P2) from the trans form to the cis form by 350 nm light irradiation.

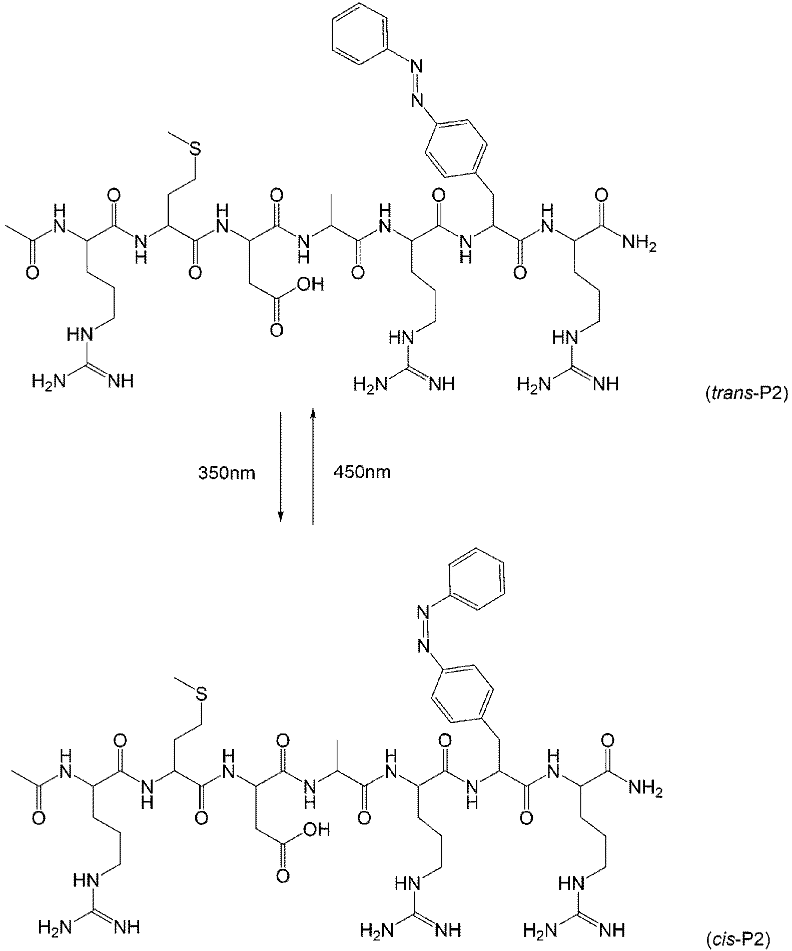

### [Example 3]

### <Synthesis Example of Compound (P3)>

The above-described compound (P3) was synthesized by solid-phase peptide synthesis. The pentafluorophenylazophenylalanine used in peptide synthesis was purchased from Tokyo Chemical Industry Co., Ltd., and the other amino acids (arginine, aspartic acid, alanine, methionine) were purchased from Watanabe Chemical Industry Co., Ltd. The synthesized compound (P3) was analyzed using matrix-assisted laser desorption/ionization (MALDI). A peak (observed value 1007.7) derived from the molecular weight (theoretical value 1007.2) of a fragment structure in which C₆F₅N was desorbed from the compound (P3) and protons were added was observed. In addition, from 470 MHz fluorine nuclear magnetic resonance spectrum (JEOL ECX-400) measurement, signals (-151.77, -152.8, -153.58 ppm) derived from the structure of the C₆F₅-N=N-C₆H₄- portion in the compound (P3) were observed. These results confirmed that the compound (P3) was obtained.

### <Photoresponsiveness of Compound (P3): UV-vis Analysis>

The compound (P3) was dispersed in a D-MEM buffer solution (pH 8.5) including 4 mM HEPES to produce a dispersion liquid including 1 wt*%* of the compound (P3). This dispersion liquid was irradiated with 450 nm or 550 nm light and the absorption spectrum was analyzed by ultraviolet-visible spectroscopy (UV-vis) (JASCO V-650 spectrophotometer). Measurements were performed at room temperature (25°C).

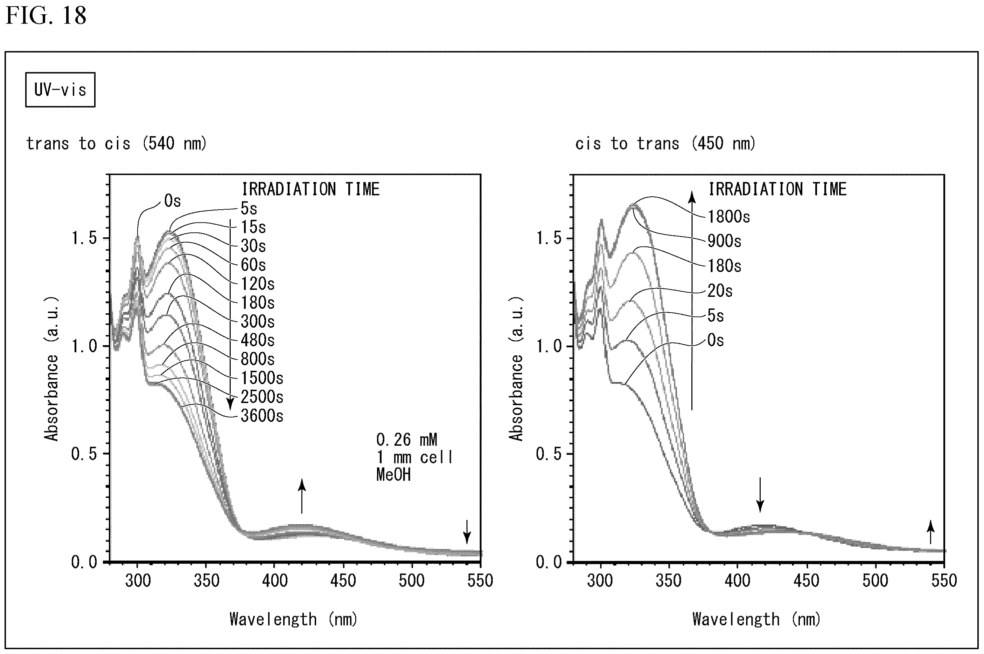

FIG. 18 shows the UV-vis analysis results. The left side of the figure shows the absorption spectrum when irradiated with 540 nm light. The right side of the figure shows the absorption spectrum when irradiated with 550 nm light.

When irradiated with 540 nm light, a decrease in the absorption peak near 320 nm and an increase in the peak near 430 nm were observed (FIG. 18, left side). These results confirmed that the azobenzene structure in the compound (P3) was photoisomerized from the trans form to the cis form. When irradiated with 450 nm light, an increase in the absorption peak near 320 nm and a decrease in the peak near 430 nm were observed (FIG. 18, right side). These results confirmed that the azobenzene structure in compound (P3) was photoisomerized from the cis form to the trans form.

The above-described results confirmed that the compound (P3) is reversibly isomerized from the trans form to the cis form and from the cis form to the trans form when irradiated with light in the visible light band of 540 nm and 450 nm.

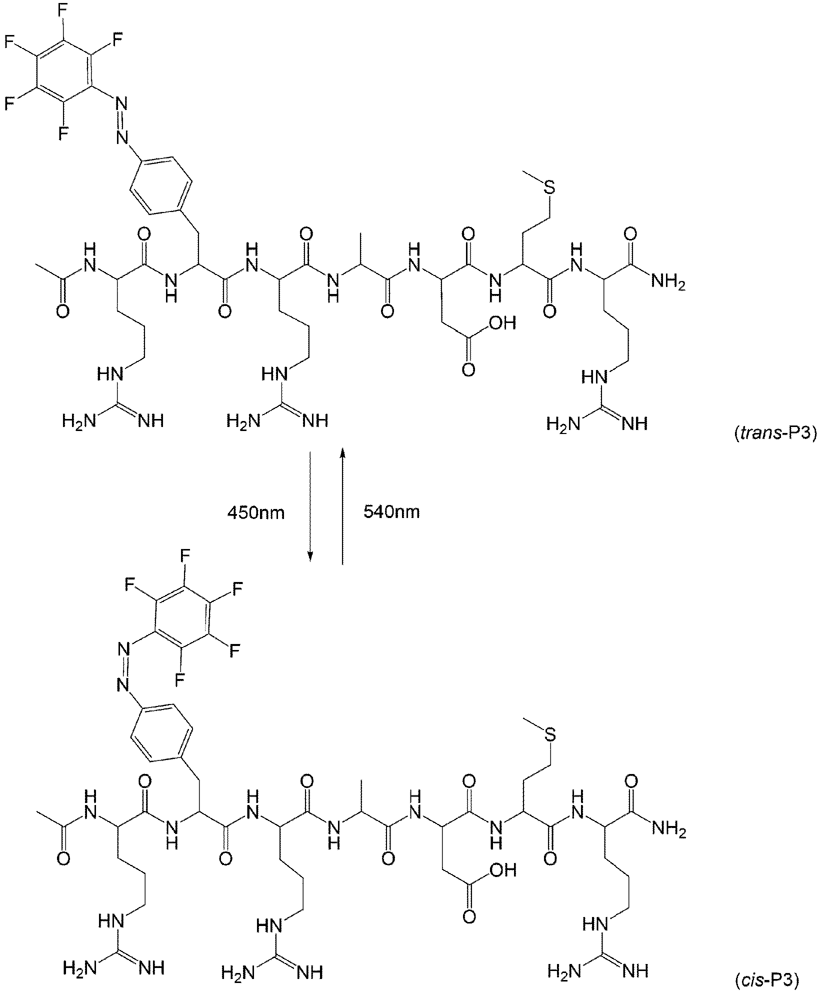

### <Photoresponsiveness of Compound (P3): IR analysis, TEM analysis>

The compound (P3) was dispersed in a D-MEM buffer solution (4 mM HEPES, containing 1 wt*%* NaHCO₃, pH 8.5) to produce a dispersion liquid including 1.0 wt*%* compound (P3). This dispersion liquid was incubated for 3 days in a dark place at 37°C in a 5*%* CO₂ atmosphere. Thereafter, the dispersion liquid was irradiated with 450 nm or 550 nm light and absorption spectrum analysis was performed by infrared absorption spectroscopy analysis (IR) (JASCO FTIR-6100) and analysis was performed using a transmission electron microscope (JEOL JEM-1400).

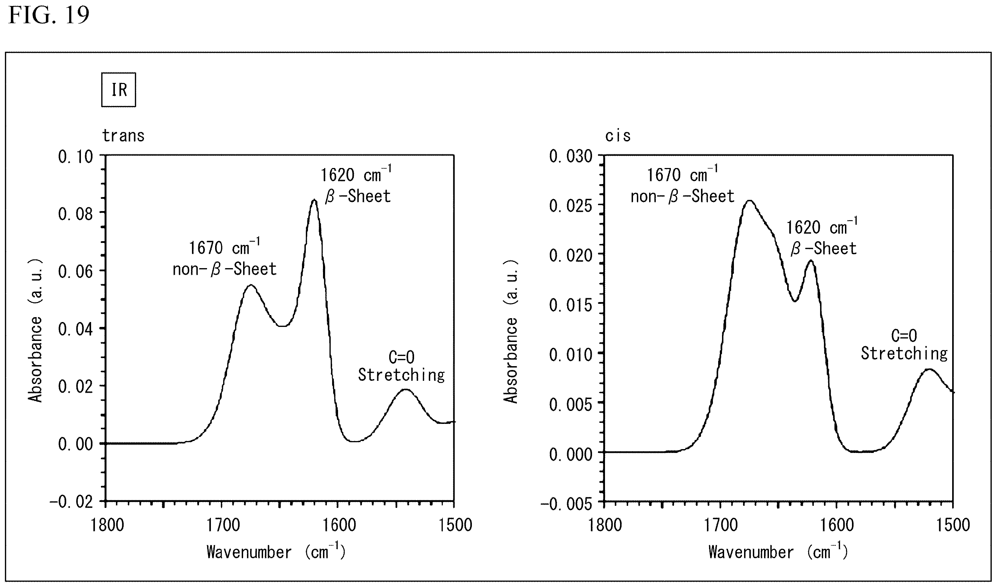

The results of the IR analysis are shown in FIG. 19. In the trans form (irradiated with 450 nm light; left side of the figure) of the compound (P3), the peak at 1670 cm⁻¹ was decreased and the peak at 1620 cm⁻¹ was increased compared to the cis form (irradiated with 540 nm light; right side of the figure). This result suggests that the compound (P3) forms β-sheets in the trans form.

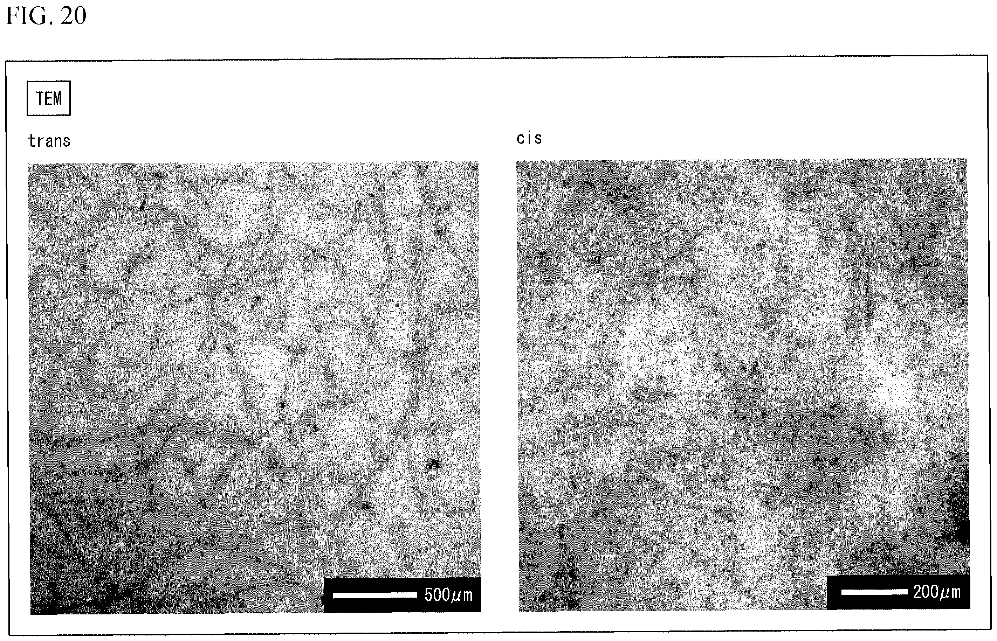

The results of the TEM analysis are shown in FIG. 20. The TEM image confirmed that the trans form (irradiated with 450 nm light; left side of the figure) of the compound (P3) forms a structure in a fibrous state. In addition, it was also confirmed that the cis form (irradiated with 540 nm light; right side of the figure) of the compound (P3) was dispersed almost evenly in the solution.

### <Rheological Evaluation>

The trans form of the compound (P3) was dispersed in a D-MEM buffer solution (4 mM HEPES, containing 1 wt*%* NaHCO₃, pH 8.5) to produce a dispersion liquid including 1.0 wt*%* of the compound (P3). This dispersion liquid was incubated for 3 days in a dark place at 37°C in a 5*%* CO₂ atmosphere. The storage modulus G' and loss modulus G" were then measured using a rotational rheometer (Malvern Panalytical, Kinexus lab+).

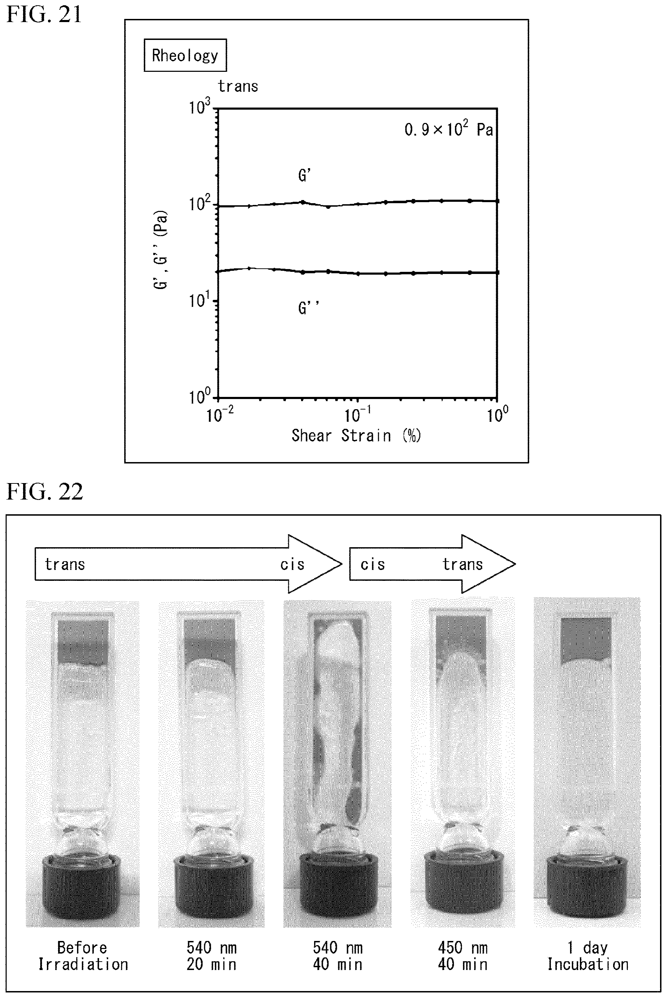

The results are shown in FIG. 21. In the trans form of the compound (P3), the storage modulus G' exhibited a value greater than the loss modulus G", confirming that a gel was formed.

### <Reversible Gel-Sol Transition>

The compound (P3) was dispersed in a D-MEM buffer solution (4 mM HEPES, containing 1 wt*%* NaHCO3, pH 8.5) to produce a dispersion liquid including 1.0 wt*%* compound (P3). This dispersion liquid was placed in a test tube to observe the changes in gelation and solation caused by light irradiation. After the start of light irradiation, incubation was performed at 37°C in a 5*%* CO₂ atmosphere.

The results are shown in FIG. 22. At the start of light irradiation (before irradiation), the compound (P3) was in the trans form and the dispersion liquid had undergone gelation. When irradiated with 540 nm light, the viscosity of the dispersion liquid decreased, and 40 minutes after the irradiation with 540 nm light, the dispersion liquid had almost completely undergone solation. This change from gel to sol was considered to be due to the trans form of the compound (P3) being converted to the cis form. Thereafter, when irradiated with 450 nm light, the dispersion liquid underwent gelation and, after one day, the dispersion liquid had almost completely undergone gelation. This change from sol to gel was considered to be due to the cis form of the compound (P3) being converted to the trans form.

### INDUSTRIAL APPLICABILITY

The present invention provides a compound useful for controlling gel-sol transition and/or droplet control, and the like. In addition, a method for producing droplets that are stable for a long time and able to be fused at any timing by using the compound is provided. Furthermore, a method for controlling droplets, a droplet production kit, and a droplet formation composition, which use the compound, as well as a reaction method using the droplets, are provided.

An explanation was given above of preferable Examples of the present invention, but the present invention is not limited to these Examples. Additions, omissions, substitutions, and other modifications to the configuration are possible in a range not departing from the gist of the present invention. The present invention is not limited by the above-described explanation, but is limited only by the scope of the attached claims.

### REFERENCE SIGNS LIST

1 First droplet
2 Second droplet
10 First channel
20 Second channel
30 Third channel

## Claims

1. A compound comprising:
a peptide structure represented by General Formula (p1) or (p2),
XaXb(XaXc)ₙXaXdXa... (p1),
XaXd(XaXc)ₙXaXbXa... (p2),
[in the formulas, Xa represents an amino acid residue having a hydrophilic side chain, Xb represents an amino acid residue having a side chain represented by General Formula (b1), Xc and Xd each independently represent an amino acid residue or a glycine residue having a hydrophobic side chain, n represents an integer of 1 to 3, a plurality of Xa's may each be identical or different, and when n is 2 or 3, a plurality of Xc's may each be identical or different,]
*-Ar²-N=N-Ar¹ (b1)
[in the formula, Ar¹ represents an aryl group or a heteroaryl group which may have a substituent, Ar² represents an arylene group or a heteroarylene group which may have a substituent, and * represents a bond.]

2. The compound according to Claim 1,
wherein Xc represents an alanine residue or a glycine residue, and Xd represents an amino acid residue having a hydrophobic side chain having 3 or more carbon atoms.

3. The compound according to Claim 1,
wherein the side chain represented by General Formula (b1) is a side chain represented by General Formula (b1-1), 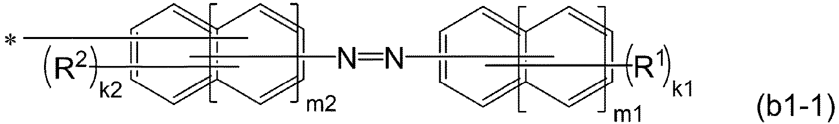
[in the formula, m1 and m2 each independently represent an integer of 0 to 3, R¹ and R² each independently represent a substituent, k1 and k2 each independently represent an integer of 0 or more as allowed by an atomic valence, when k1 is an integer of 2 or more, two or more R¹'s may each be identical or different, when k2 is an integer of 2 or more, the two or more R²'s may each be identical or different, and * represents a bond.]

4. The compound according to Claim 1,
wherein the peptide structure is a peptide structure represented by General Formula (p1-1) or (p2-1), 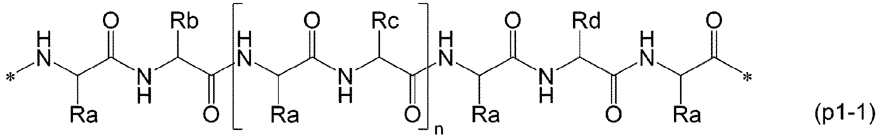 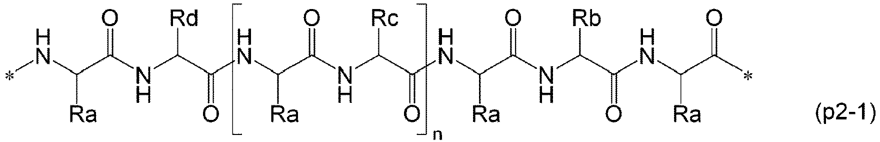
[in the formulas, Ra represents a hydrophilic side chain, Rb represents a side chain represented by General Formula (b1), Rc and Rd each independently represent a hydrophobic side chain or a hydrogen atom, n represents an integer of 1 to 3, a plurality of Ra's may each be identical or different, when n is 2 or 3, the plurality of Ra's may each be identical or different, and * represents a bond.]

5. The compound according to Claim 4,
wherein Rc represents a hydrogen atom or a methyl group, and Rd represents a hydrophobic side chain having 3 or more carbon atoms.

6. The compound according to Claim 1, which is a peptide consisting of 40 or fewer amino acid residues.

7. The compound according to Claim 1, further comprising:
a peptide structure represented by Formula (p1-1-1), (p2-1-1), (p1-1-2), or (p2-1-2), 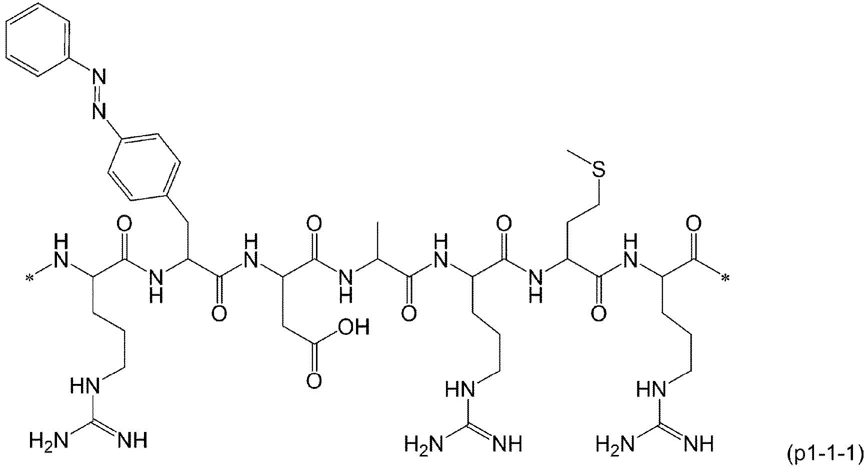 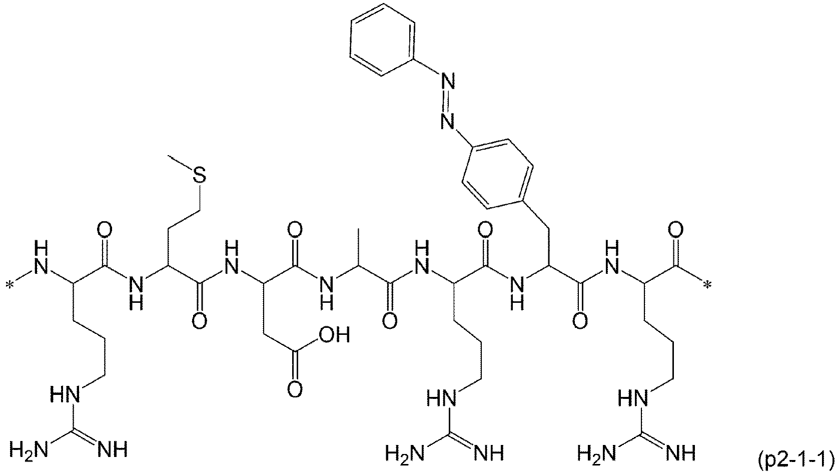 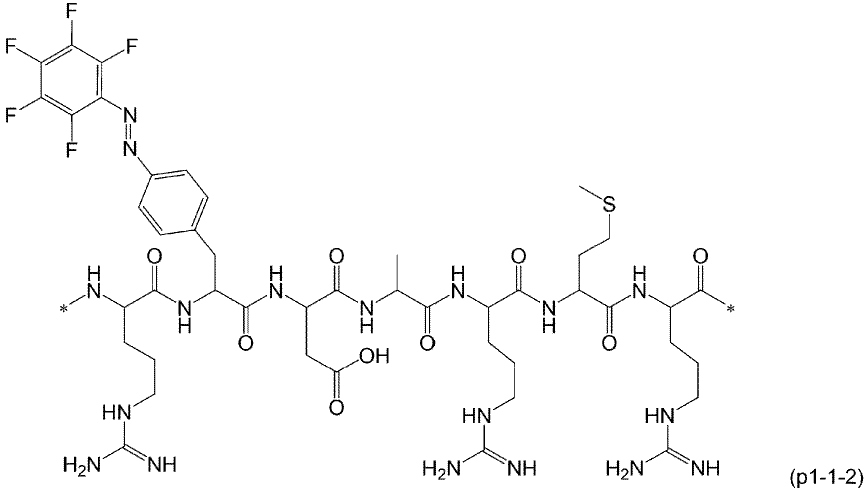 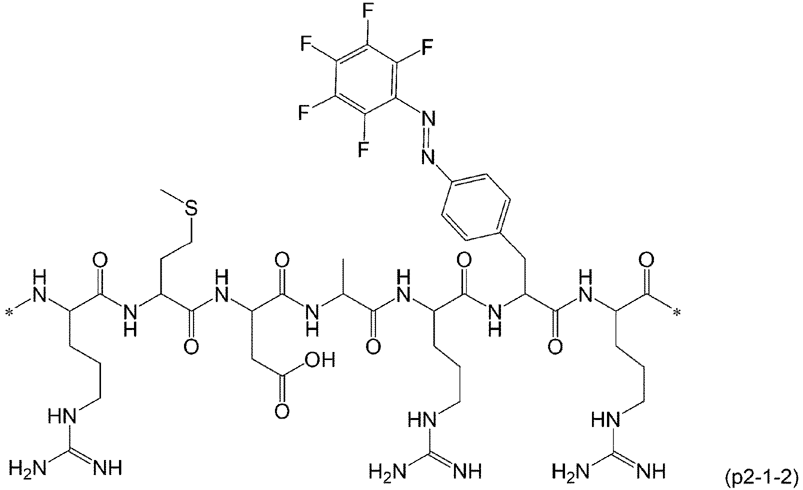
[* is a bond bonded to an adjacent atom.]

8. The compound according to Claim 1, which is represented by any of Formulas (P1) to (P4).

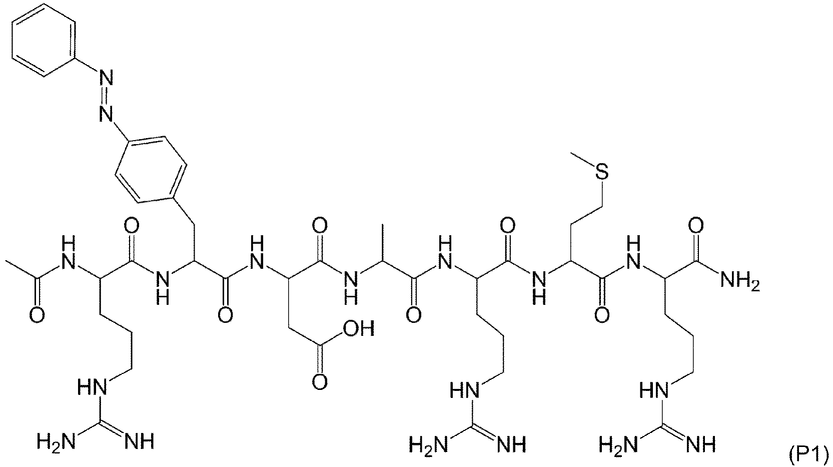

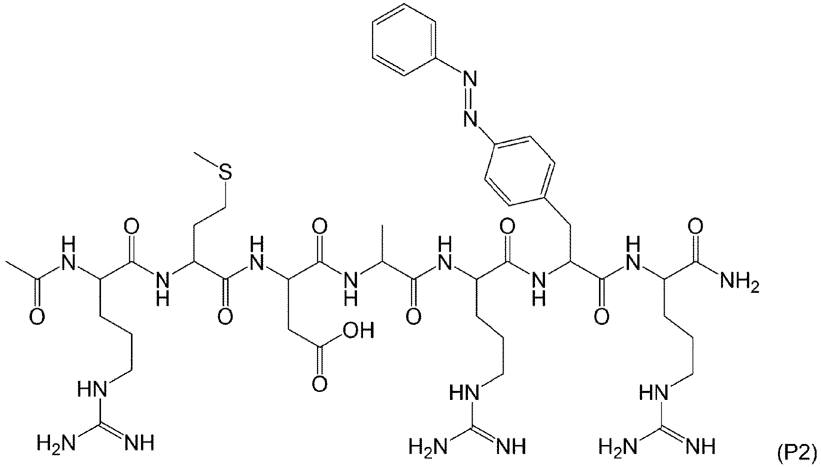

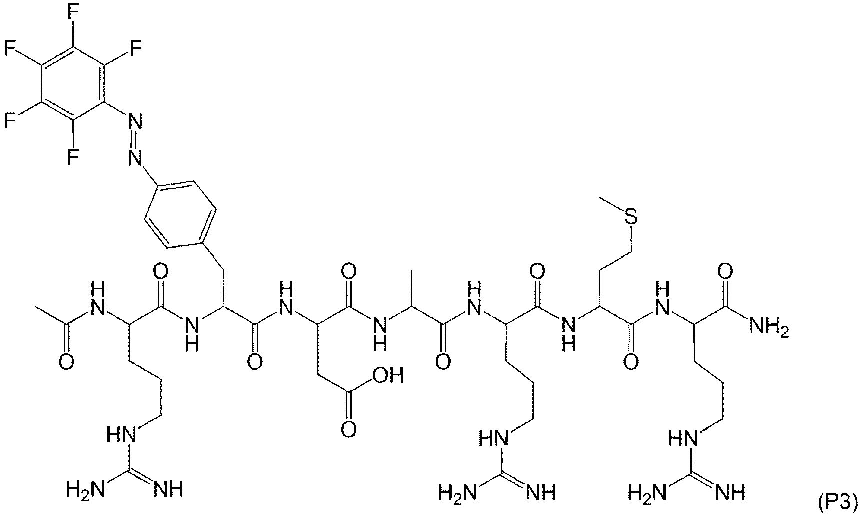

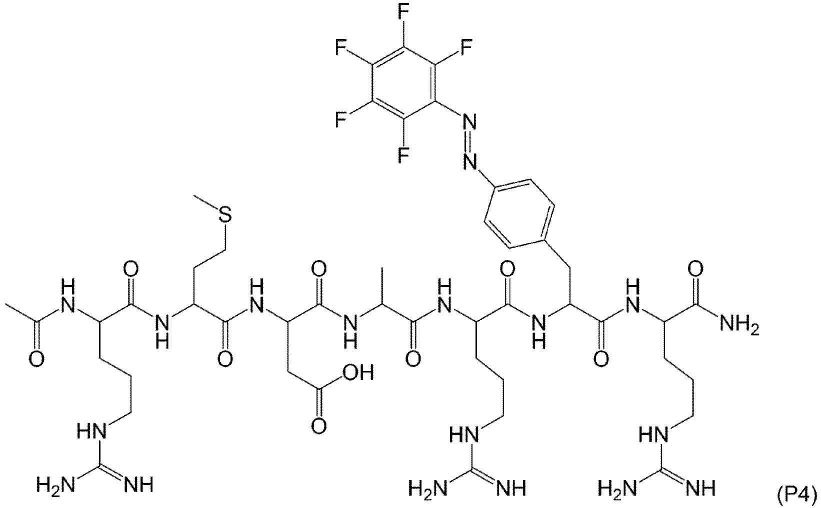

9. A gelling agent comprising:
the compound according to any one of Claims 1 to 8.

10. A droplet production kit comprising:
the compound according to any one of Claims 1 to 8;
a polysaccharide; and
a polyether.

11. A method for producing droplets, the method comprising:
forming droplets by stirring a solution including the compound according to any one of Claims 1 to 8, a polysaccharide, and a polyether.

12. The method for producing droplets according to Claim 11, the method further comprising:
irradiating the formed droplets with light.

13. A reaction method comprising:
(a) a step of stirring a solution including the compound according to any one of Claims 1 to 8, a polysaccharide, a polyether, and a first reactant to produce a first droplet including the first reactant;
(b) a step of stirring a solution including the compound according to any one of Claims 1 to 8, a polysaccharide, a polyether, and a second reactant to produce a second droplet including the second reactant;
(c) a step of mixing the first droplet and the second droplet; and
(d) a step of irradiating the mixed first droplet and second droplet with light to fuse the first droplet and the second droplet to produce a reaction site droplet including the first reactant and the second reactant.

14. The reaction method according to Claim 13, further comprising:
(e) a step of incubating the reaction site droplet.

15. A droplet formation composition comprising:
the compound according to any one of Claims 1 to 8;
a polysaccharide; and
a polyether.

16. A droplet control agent comprising:
the compound according to any one of Claims 1 to 8.

17. A method for controlling droplets, the method comprising:
causing droplets formed by stirring a solution including a polysaccharide and a polyether to coexist with the compound according to any one of Claims 1 to 8.

18. The method for controlling droplets according to Claim 17, the method further comprising:
irradiating the droplets coexisting with the compound with light.
